# EUROPEAN PATENT APPLICATION

(11) **EP 4 331 673 A1**
(43) Date of publication of application: **06.03.2024**
(21) Application number: 22795890.7
(22) Date of filing: 28.04.2022
(51) Int. Cl.: A61P 27/02, A61K 9/10, C12M 3/00, C12N 5/071, C12N 5/074, C12N 1/00, A61K 35/30, A61K 35/545, A61L 27/38, A61L 27/54

(54) **CORD-LIKE AGGREGATES OF RETINAL PIGMENT EPITHELIAL CELLS, DEVICE AND PRODUCTION METHOD FOR PRODUCING SAME, AND THERAPEUTIC AGENT COMPRISING SAID CORD-LIKE AGGREGATES**

(30) Priority: 30.04.2021 JP 2021078154
(71) Applicant: RIKEN, Wako-shi, Saitama 351-0198 (JP)
(72) Inventor: TAKAHASHI, Masayo, Wako-shi, Saitama 351-0198 (JP); TANAKA, Yuji, Wako-shi, Saitama 351-0198 (JP); TANAKA, Yo, Wako-shi, Saitama 351-0198 (JP); TANAKA, Nobuyuki, Wako-shi, Saitama 351-0198 (JP); AMAYA, Satoshi, Wako-shi, Saitama 351-0198 (JP); NISHIDA, Mitsuhiro, Wako-shi, Saitama 351-0198 (JP); MANDAI, Michiko, Wako-shi, Saitama 351-0198 (JP)
(74) Representative: Weickmann & Weickmann PartmbB
(86) International application number: PCT/JP2022/019293
(87) International publication number: WO 2022/230977

(57) **Abstract**

The present invention provides a transplant material of a retinal pigment epithelial cell that can be prepared in a short time at a low cost, and that can be easily controlled at the site of transplantation even though the transplantation method is non-invasive, and a method for producing such transplant material.

## Description

### [Technical Field]

The present invention relates to string-like aggregates of retinal pigment epithelial cells, devices and production methods for producing same, and therapeutic drugs containing the string-like aggregates.

### [Background Art]

The use of ES/iPSC-derived retinal pigment epithelial cells (RPE) for cell-based regenerative therapy for ophthalmic diseases has been one of the major interests in the clinical field of ophthalmology for the past 10 years (Non Patent Literature 1). These clinical studies have demonstrated the safety of this therapeutic approach, with some possible efficacy. So far, two approaches of an approach using cell suspension and an approach using RPE sheet have been used in these clinical studies, both of which have advantages and disadvantages. Transplantation using cell suspensions can utilize cell stocks immediately from tubes or cell stocks obtained by short-term culture, and transplantation is possible with minimal surgical invasiveness. However, it is difficult to control the arrangement of cells at the transplant site, and the transplanted cells often form an epiretinal membrane (ERM) in the vitreous body (Non Patent Literatures 1, 2). In contrast, RPE sheets permit visual confirmation that the grafted sheet has been placed by an accurate method as desired. However, preparation of sheet requires time, cost, and an invasive surgical procedure involving large incisions in the sclera and neural retina (Non Patent Literature 3). Therefore, the problem of providing a transplant material of a retinal pigment epithelial cell that can be prepared in a short time at a low cost, and that can be easily controlled at the site of transplantation even though the transplantation method is non-invasive remains unresolved.

### [Citation List]

### [Non Patent Literature]

[NPL 1]
   Sugita, S et al., (2020) J. Clin. Med. 9, 2217.
[NPL 2]
   Schwartz, S.D. et al., (2015) Lancet 385, 509-516.
[NPL 3]
   Lyndon da Cruz et al., Nature Biotechnology Advance Online Publication, published online 19 March 2018.

### [Summary of Invention]

### [Technical Problem]

The problem to be solved by this invention is provision of a transplant material of a retinal pigment epithelial cell that can be prepared in a short time and at low cost, and that can be easily controlled at the site of transplantation even though the transplantation method is non-invasive, and a method for producing such transplant material.

### [Solution to Problem]

The present inventors hypothesized that even string-like aggregates of RPE cells that can be easily injected subretinally can be expanded to cover a certain area, similar to a transplantation of a small sheet. In recent years, the relationship among surface coatability, tissue contraction, and adhesive strength has been reported as a morphological mechanism of microtissues (Yamashita, T. et al., (2016). Acta Biomater. 45, 85-97.). The present inventors confirmed that cells with well-balanced adhesive force and contractile force spontaneously generated spheroid-like tissues on concave surfaces with small curvature. Based on this finding, they investigated the curvature of the lower end of the concave surface and found that groove surfaces with small curvature cause generation of band-like tissues from the cells. The generation of small tissues basically requires special techniques such as photolithography. They introduced a production technique that combines polydimethyl siloxane (PDMS) soft lithography and simple three-dimensional (3D) printing, making this tissue engineering approach widely possible. In the present invention, they produced a PDMS-based culture device with narrow grooves and investigated whether hiPSC-RPE cells could form string-like aggregates. They then confirmed whether hiPSC-RPE cells could expand from the string-like aggregates plated on the dish and whether the expanded hiPSC-RPE cells could exhibit RPE characteristics similar to those before formation of the string-like aggregates. They have also verified whether hiPSC-RPE that had formed string-like aggregates could be practically injected into the eyes of animals.

That is, the present invention relates to the following.

[1] A string-like aggregate of retinal pigment epithelial cells.
[2] The string-like aggregate of the above-mentioned [1], having a ratio of total length/outer diameter of body of 2 to 1,000.
[3] The string-like aggregate of the above-mentioned [2], having a circular or ellipse shape in the cross sectional shape.
[4] The string-like aggregate of any of the above-mentioned [1] to [3], wherein the retinal pigment epithelial cell is a cell induced to differentiate from a pluripotent stem cell.
[5] A device for producing the string-like aggregate of any of the above-mentioned [1] to [4], comprising a base member, wherein the base member has a top surface provided with one or more grooves, and the groove has a cavity part with a length and a width of the string-like aggregate, as a mold for culturing retinal pigment epithelial cells to be seeded into a string-like aggregate.
[6] The device of the above-mentioned [5], wherein the aforementioned groove has a cavity part as the bottom of the groove, and a top part located on the cavity part, wherein the top part has an opening of the aforementioned groove.
[7] The device of the above-mentioned [5] or [6], wherein the aforementioned groove is V-shaped in the cross sectional shape, and the deepest part of the V-shape is rounded.
[8] A method for producing a string-like aggregate of retinal pigment epithelial cells, comprising the following steps:
   (1) a step of seeding retinal pigment epithelial cells suspended in a liquid medium in the groove of the device of any of the above-mentioned [5] to [7],
   (2) a step of culturing the retinal pigment epithelial cells in the aforementioned groove to form a string-like aggregate of the retinal pigment epithelial cells.
[9] The method of the above-mentioned [8], wherein the medium comprises a ROCK inhibitor.
[10] The method of the above-mentioned [9], wherein the ROCK inhibitor is Y-27632.
[11] The method of any of the above-mentioned [8] to [10], wherein the retinal pigment epithelial cells cultured in the groove has a density of 2.5×10³ cells/mL to 5×10⁵ cells/mL.
[12] The method of any one of the above-mentioned [8] to [11], wherein the retinal pigment epithelial cells are cultured for a period of one day to 7 days.
[13] A pharmaceutical composition comprising the string-like aggregate of retinal pigment epithelial cells of any of the above-mentioned [1] to [4].
[14] A therapeutic drug for a disease caused by a disorder of a retinal pigment epithelium, comprising the string-like aggregate of retinal pigment epithelial cells of any of the above-mentioned [1] to [4].

### [Advantageous Effects of Invention]

According to the present invention, it is possible to produce a transplant material of a retinal pigment epithelial cell that can be prepared in a short time at a low cost, and that can be easily controlled at the site of transplantation even though the transplantation method is non-invasive.

### [Brief Description of Drawings]

[Fig. 1]

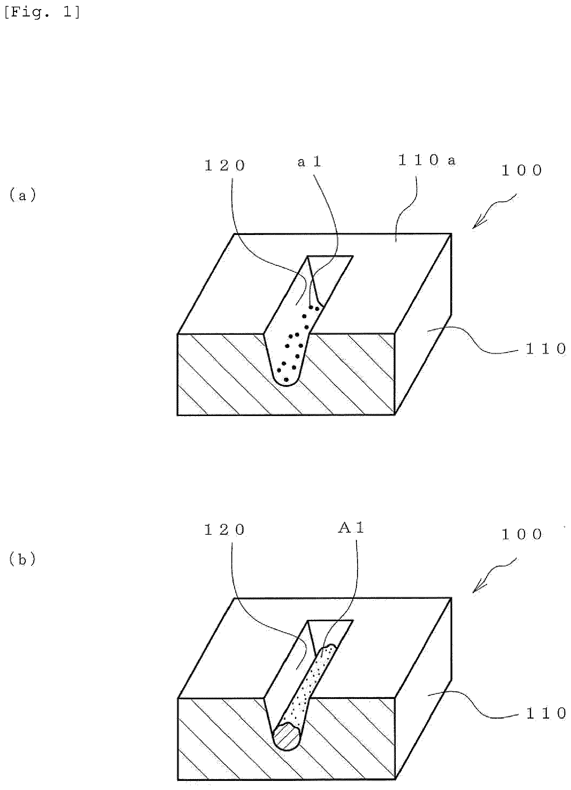

   Fig. 1 is a perspective view illustrating the structure of the device of the present invention and a method for producing a string-like aggregate using the same. The Figure shows an embodiment in which a single groove is provided on the top surface of the base member, in order to explain the groove in an easy-to-understand manner. Further, in order to show the cross section of the groove (the cross section when the groove is cut perpendicularly to its longitudinal direction), only a part of the total length of the groove is shown. Fig. 1(a) shows a state in which retinal pigment epithelial cells are seeded in the groove together with a liquid medium (not shown). Fig. 1(b) shows the state in which the seeded retinal pigment epithelial cells have grown into a string-like aggregate in the groove.
[Fig. 2]

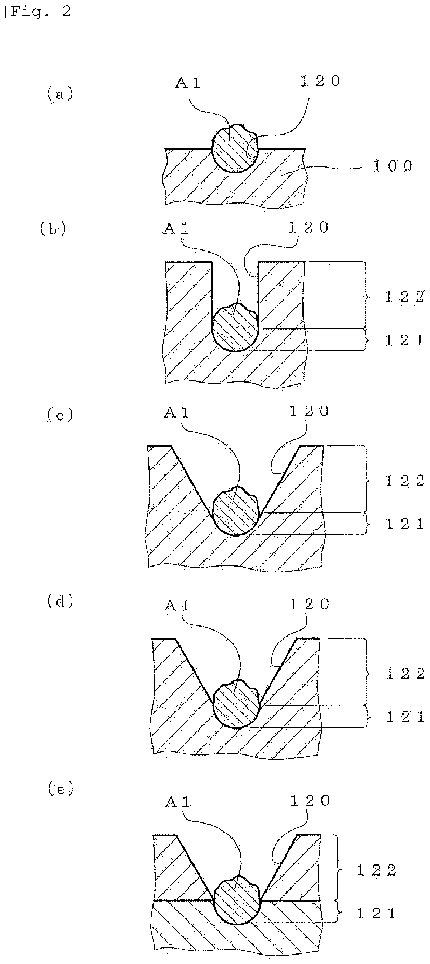

   Fig. 2 shows diagrams illustrating the shapes of the groove and cavity part.
[Fig. 3]

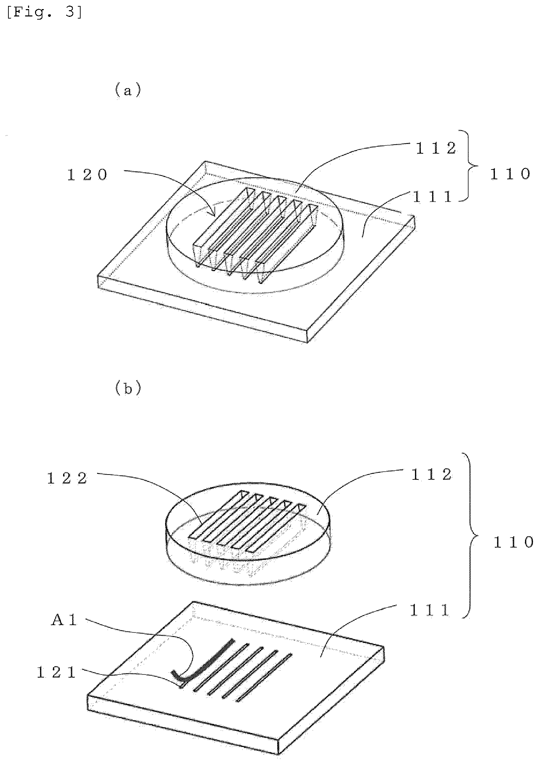

   Fig. 3 shows diagrams illustrating an example of a preferred embodiment of the groove and cavity part.
[Fig. 4]

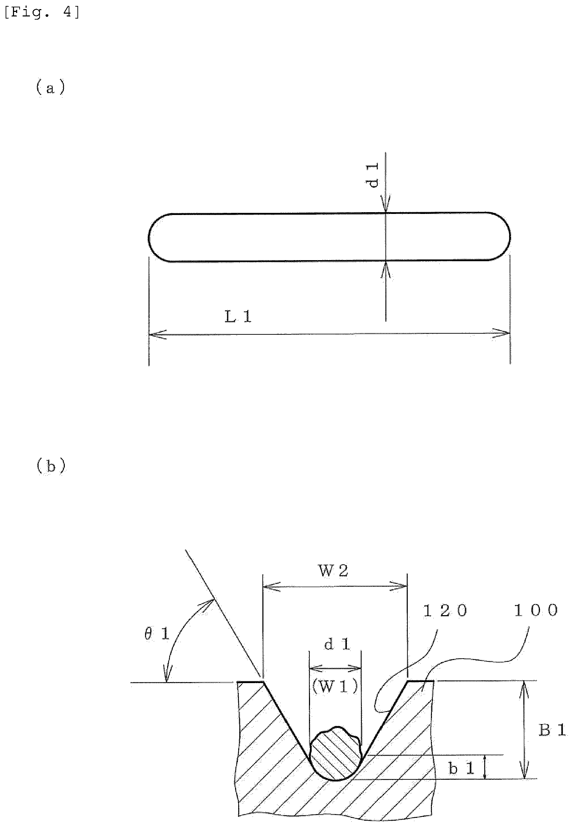

   Fig. 4 shows schematic diagrams explaining the size of each part of the string-like aggregate, constitution of the device of the present invention, and the relationship between the string-like aggregate and the device. For explanation, Fig. 4(a) is a diagram simply showing the overall shape of the string-like aggregate. Fig. 4(b) is a diagram showing one embodiment of the size relationship between the string-like aggregate formed in a groove and the groove.
[Fig. 5]

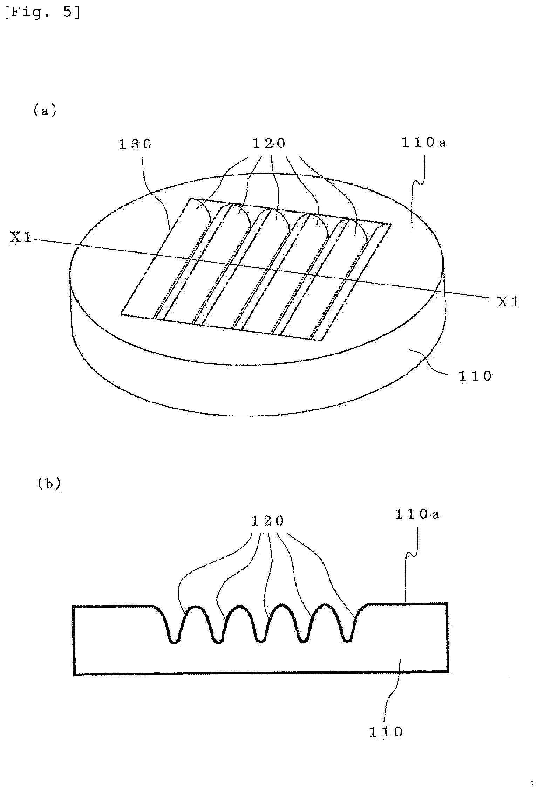

   Fig. 5 shows diagrams illustrating a preferred embodiment of said device of the present invention. Fig. 5(a) is a perspective view showing the entire device, and Fig. 5(b) is a cross sectional view of the device shown in Fig. 5(a) taken along the cutting line X1-X1 extending in the width direction of the groove in the thickness direction of the device. Hatching on the cut plane is omitted.
[Fig. 6]

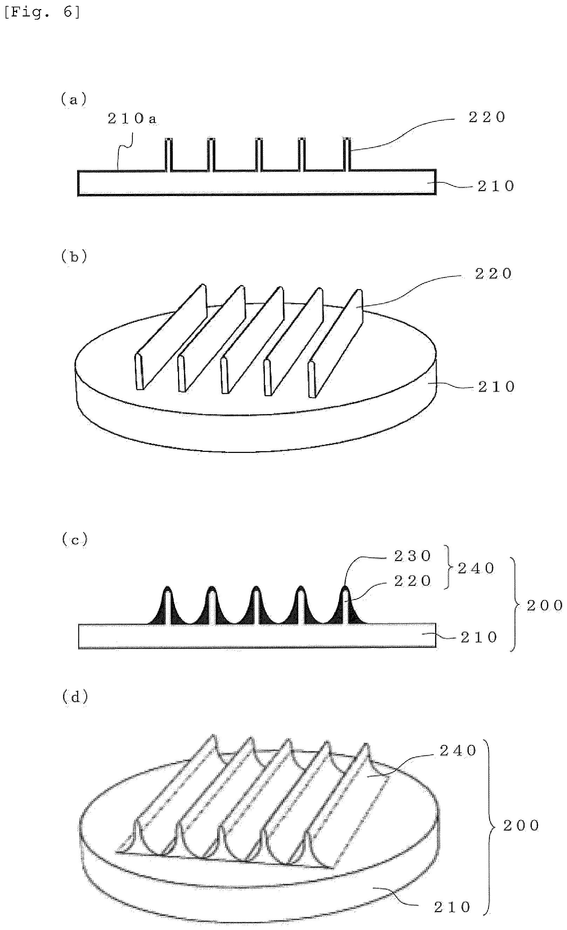

   Fig. 6 shows diagrams illustrating one embodiment of a forming mold for producing the device of the present invention, and also a diagram illustrating one embodiment of a method for producing the forming mold. Fig. 6(b) is a perspective view of the mold component shown in Fig. 6(a). Fig. 6(d) is a perspective view of the mold shown in Fig. 6(c).
[Fig. 7]

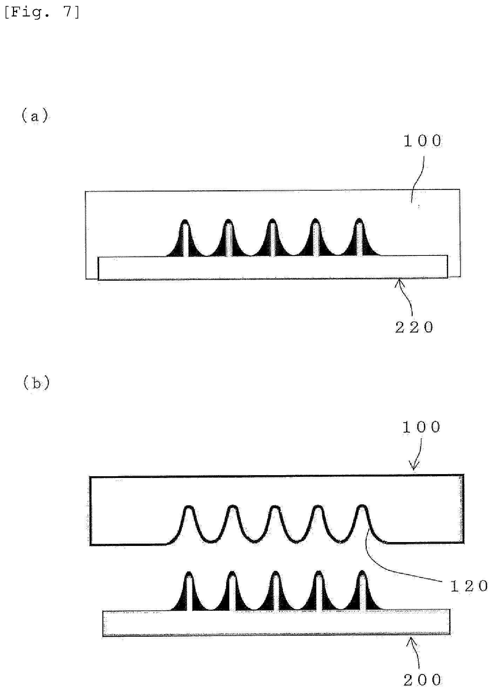

   Fig. 7 shows sectional views illustrating one embodiment of the production step of the device of the present invention, schematically showing that said device is resin-molded using the forming mold shown in Figs. 6(c) and 6(d).
[Fig. 8]

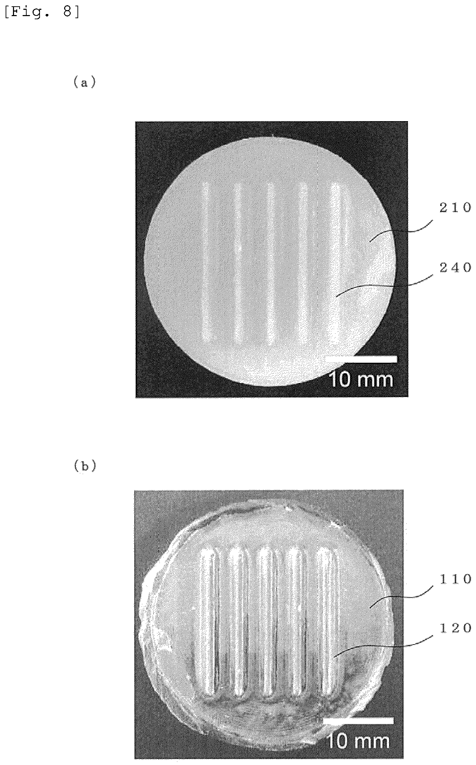

   Fig. 8 is a photographic diagram (Fig. 8(a)) showing a forming mold actually produced in an example of the present invention, and a photographic diagram (Fig. 8(b)) showing said device actually molded using the forming mold.
[Fig. 9]

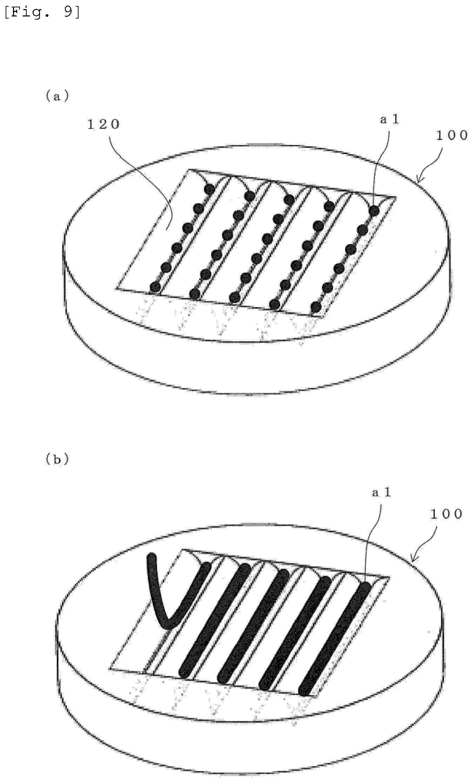

   Fig. 9 shows schematic diagrams illustrating the production method of the string-like aggregate in the present invention. Fig. 9(a) shows a state in which retinal pigment epithelial cells are seeded in the groove of the device shown in Fig. 5 (liquid medium is not shown), and Fig. 9(b) schematically shows how a string-like aggregate formed in the groove is taken out from the groove.
[Fig. 10]

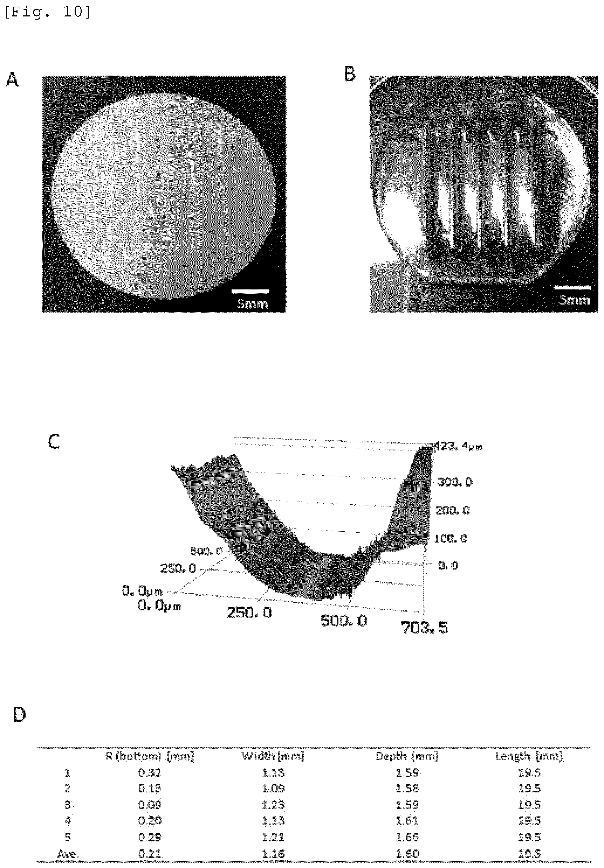

   Fig. 10 shows the measurement results of a PDMS groove structure. A: photograph of a PDMS-coated mold with 5 fins (length 19.5 mm and height 1.6 mm). B: photograph of a PDMS-based culture device for groove structure measurement with 5 grooves (length 19.5 mm, width 1 mm, and depth 1.6 mm). C: Image of the groove bottom measured using a laser microscope. D: Measurement of the groove shape of a PDMS-based culture device. The average values for each measurement item are bottom surface radius of curvature 0.21 mm, groove width 1.16 mm, groove depth 1.60 mm.
[Fig. 11]

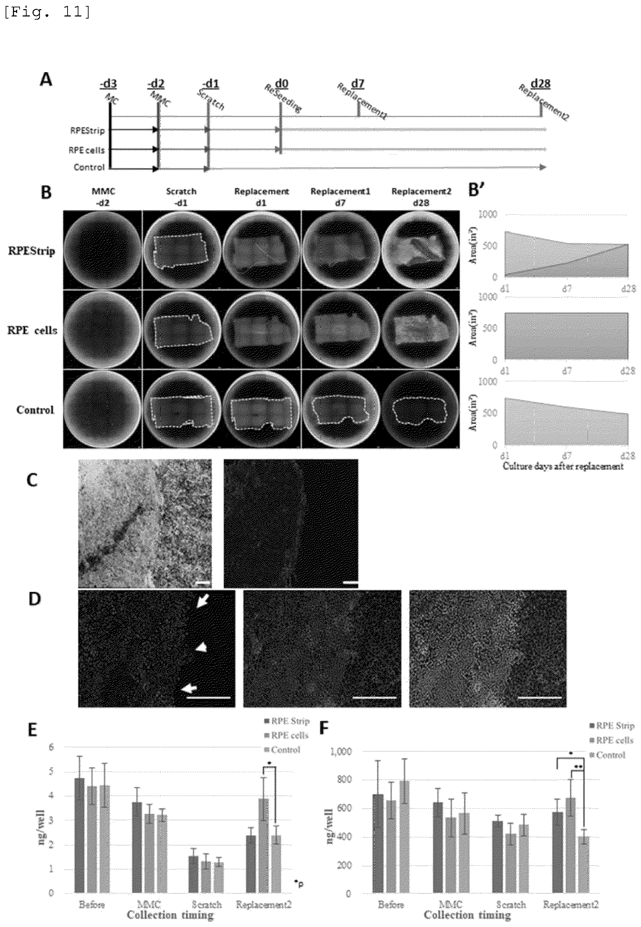

   Fig. 11 shows that string-like aggregates partially restored function as they replaced damaged RPE-deficient regions. A: In vitro disease model and transplantation experiment schedule. For pathological RPE degeneration/disorder state, the transplantation step was mimicked in vitro by treating RPE cells with MMC and creating a scratch, followed by plating either hiPSC-RPE cell suspension or string-like aggregate (201B7modFucci line) onto the deficient region. B: Expansion of plated hiRPE cells on the RPE-deficient region was monitored by mCherry expression by cells in the G0-G1 cell cycle of the 201B7-Fucci hiPSC line. The initially scratched region and the defective region in the control wells are indicated by dotted lines. B': Panels show RPE-deficient regions after scratching and refilled region with RPE cell expansion from RPE cell suspension or string-like aggregate. C: RPE cells expanded from string-like aggregates appeared to stop expanding upon contact with resident RPE, which showed increased expression of mCherry along the border. D: The boundary between existing MMC-treated RPE and newly plated RPE was reasonably identified by mCherry expression and RPE cell size (arrow). MMC-treated RPEs were generally larger than RPEs expanded from string-like aggregates. ZO-1 expression was observed between resident MMC-treated population and cells within each population of freshly plated/expanded RPE cells, and also between cells at the border part. E and F: secretion of PEDF and VEGF in each well at start (-d3), after MMC (-d2), after scratching (-d1), and after presence or absence of plating of hiPSC-RPE cells or string-like aggregates (n=3). Secretion of both PEDF and VEGF decreased after MMC treatment and scratching. Without RPE cell supplementation, PEDF continued to decrease. With supplementation of RPE cells, PEDF was obtained more rapidly either in suspension or string-like aggregates.
[Fig. 12]

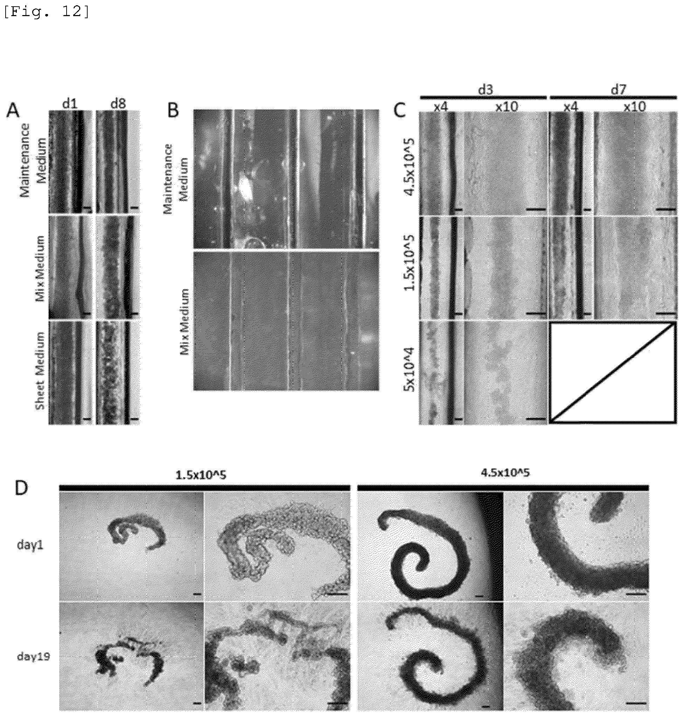

   Fig. 12 shows optimization of string-like aggregate formation. A: hiPSC-RPE cells were plated in the grooves of mold, using three types of media (sheet medium, mix medium, maintenance medium): 10 µM Y-27632. B: Appearance of string-like aggregates in the mold (mix medium, maintenance medium). C: hiPSC-RPE cells were plated at 4.5×10⁵, 1.5×10⁵, 5×10⁵ in grooves and incubated for 3 and 7 days. D: Appearance of string-like aggregates formed with 150000 or 450000 cells and left on a plate for 19 days. The string-like aggregates formed with 150000 cells lost their initial shape more rapidly than the string-like aggregates formed with 450000 cells, and almost lost their initial shape after 19 days. Scale bar, 200 µm.
[Fig. 13]

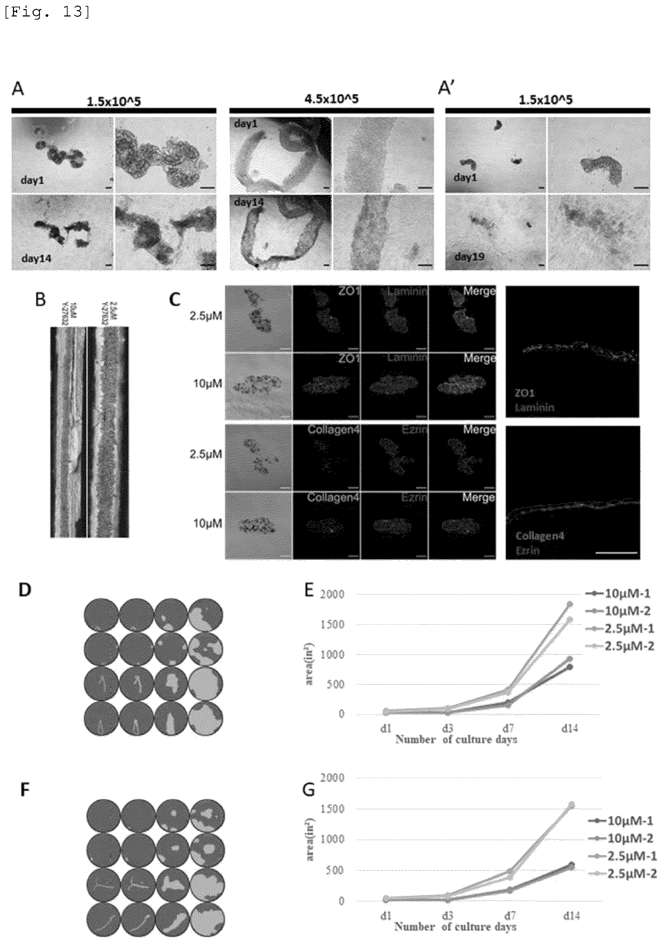

   Fig. 13 shows the influence of starting cell number and Y-27632 concentration on string-like aggregate formation. A: String-like aggregates formed with 150000 or 450000 RPE cells on day 4 were placed on a dish and appearance was photographed the next day and 14 days later. RPE cells expanded from each string-like aggregate, and string-like aggregates formed with 15000 cells lost their shape after 14 days, whereas string-like aggregates formed with 450000 cells maintained their initial shape. A': The fragmented string-like aggregates almost lost their initial shape after cell expansion. B: Appearance of string-like aggregates on day 2 after seeding RPE (2×10⁵). C: Cross section of string-like aggregates formed with either 10 µM or 2.5 µM Y-27632. Ezrin and laminin expression was fragmented, but ZO-1 was expressed intercellularly, and apical-basal polarity is not differentiated. Right panels show control staining of RPE sheets. D, E, F, G: The string-like aggregates formed with 2.5 µM Y-27632 showed higher adhesiveness to the plate, covered more area, and began to expand faster on the plate (D, E: day 2 string-like aggregates, F, G: day 3 string-like aggregates), as compared with those formed with 10 µM Y-27632.
[Fig. 14]

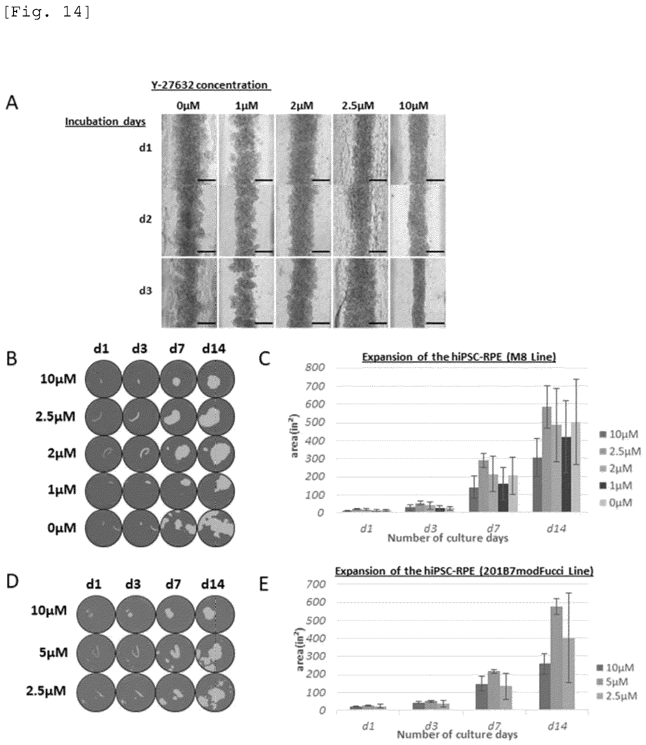

   Fig. 14 shows optimization of Y-27632 concentration for string-like aggregate formation. A: String-like aggregate formation under different Y-27632 concentrations. B: String-like aggregates (M8 line) on day 2 were plated in a 24-well plate, and the expansion of the cover region by hiPSC-RPE cells was monitored. String-like aggregates added with 2-2.5 µM Y-27632 showed highest adhesiveness to the plate and tended to adhere flat when placed on the wells. C: Expansion of hiPSC-RPE cells (M8 line). Coated region after placing string-like aggregates in each well (n=6 for each Y-27632 concentration). D: String-like aggregates (201B7modFucci line) on day 2 were placed in a 24-well plate, and the expansion of the region coated by hiPSC-RPE cells was monitored. String-like aggregates added with 2-2.5 µM Y-27632 were consistently adherent to the plate and tended to adhere flat when placed over the well. E: Expansion of hiPSC-RPE cells (201B7modFucci line). Covered area after placing string-like aggregates in each well (n=2 for each Y-27632 concentration).
[Fig. 15-1]

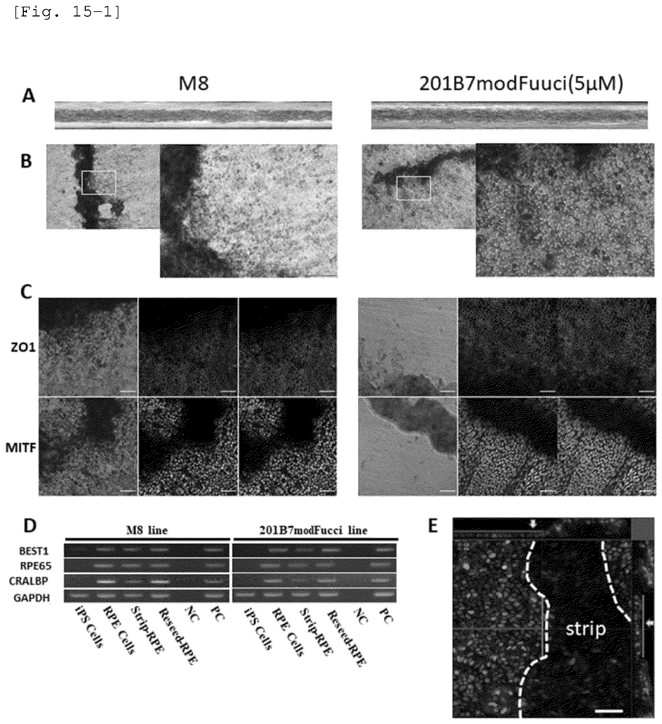

   Fig. 15-1 shows reproducibility of string-like aggregate formation using different hiPSC lines (M8 and 201B7modFucci L). A: String-like aggregates were similarly formed using different hiPSC lines, but optimization of Y-27632 was required for each line. 201B7modFucci line required 5 µM Y-27632 to stably form string-like aggregates. B: Respective string-like aggregates formed with M8 and 201B7modFucci lines adhered to the plate, were well expanded, and exhibited characteristic cobblestone appearance and dye deposition of RPE. C: RPE cells expanded from string-like aggregates expressed the tight junction marker ZO-1 and the RPE marker MiTF. D: RPE marker gene expression by hiPSC-RPE cells before string-like aggregate formation, RPE cells in string-like aggregates, and cells after expansion from string-like aggregates derived from each hiPSC line. E: RPE cells migrated and proliferated from string-like aggregates into a single layer.
[Fig. 15-2]

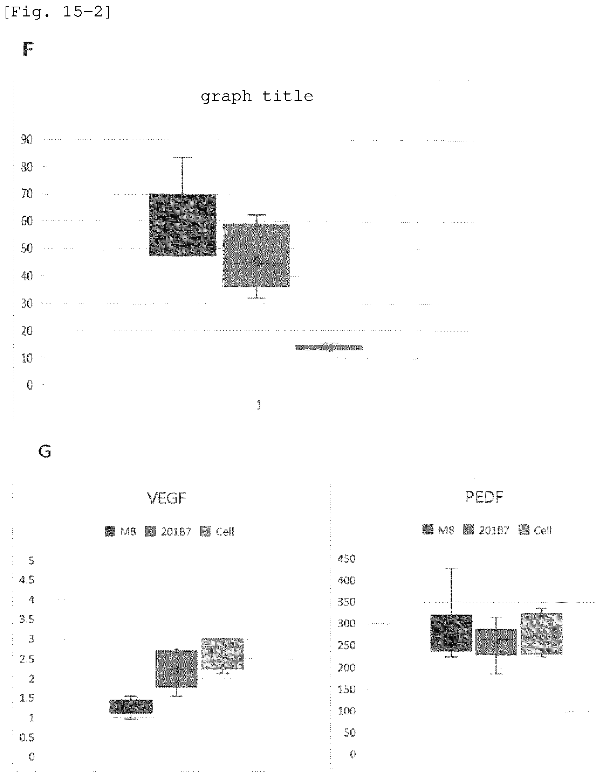

   Fig. 15-2 shows reproducibility of string-like aggregate formation using different hiPSC lines (M8 and 201B7modFucci L). F: Number of RPE cells in string-like aggregates and number of RPE cells expanded from string-like aggregates. G: RPE cells expanded from string-like aggregates secreted VEGF and PEDF. Scale bar, 50 µm (C, E).
[Fig. 16]

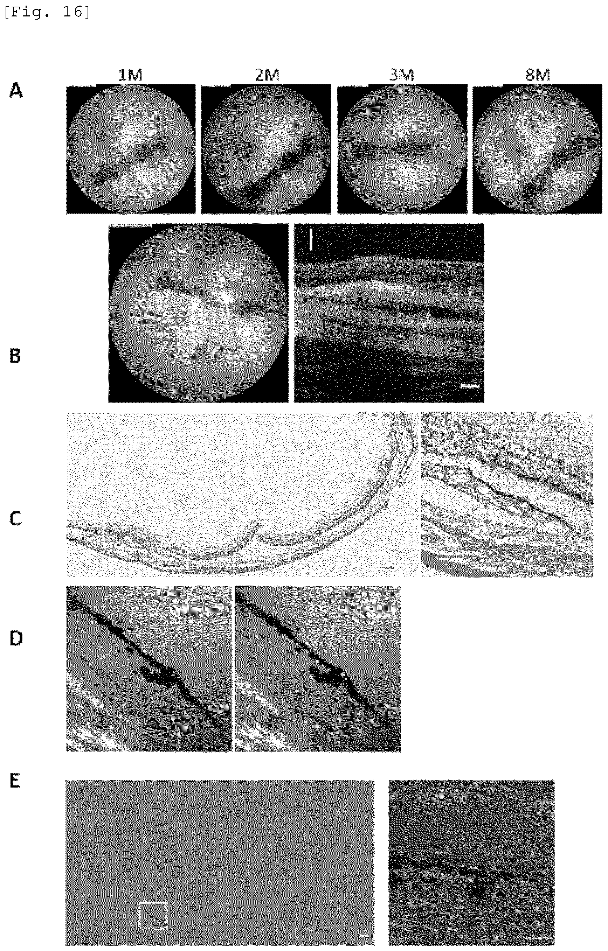

   Fig. 16 shows string-like aggregate transplantation in nude rats. A: Photographs of fundus oculi of nude rat eye after transplantation of string-like aggregates. B: Photographs of fundus oculi of nude rat eye on day 93 after transplantation of string-like aggregates. A strong RPE-like signal was observed at the transplant site by SLO-OCT. C: Hematoxylin and eosin staining of the eye after transplantation. D: Human nuclear antigen (HuNu) was positive only in the pigment sheet layer. E: Transplanted RPE exhibited well orientation as indicated by human-specific ezrin and collagen type IV which are the polar markers. Scale bar, 50 µm (B) 20 µm (E).
[Fig. 17]

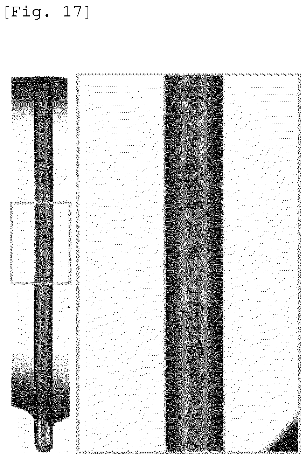

   Fig. 17 shows string-like aggregate loaded into a 24G intravenous cannula.
[Fig. 18]

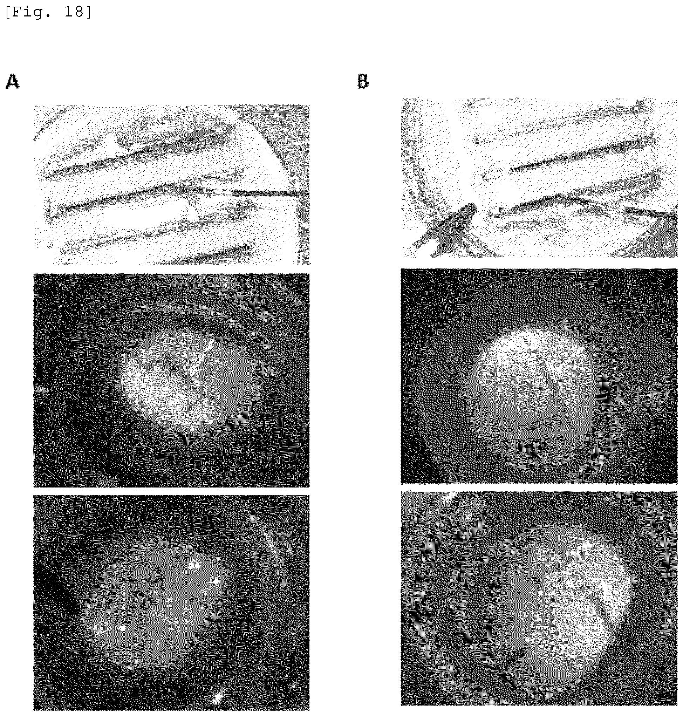

   Fig. 18 shows transplantation of string-like aggregates into two rabbit eyes. The practical handling of string-like aggregates during transplant surgery was tested in two rabbit eyes (A and B). Top: Loading of string-like aggregates. Center: String-like aggregates were slowly injected inside the retinal hemorrhage. The retinal incision site is indicated with a white arrow. Bottom: Injected string-like aggregates.
[Fig. 19]

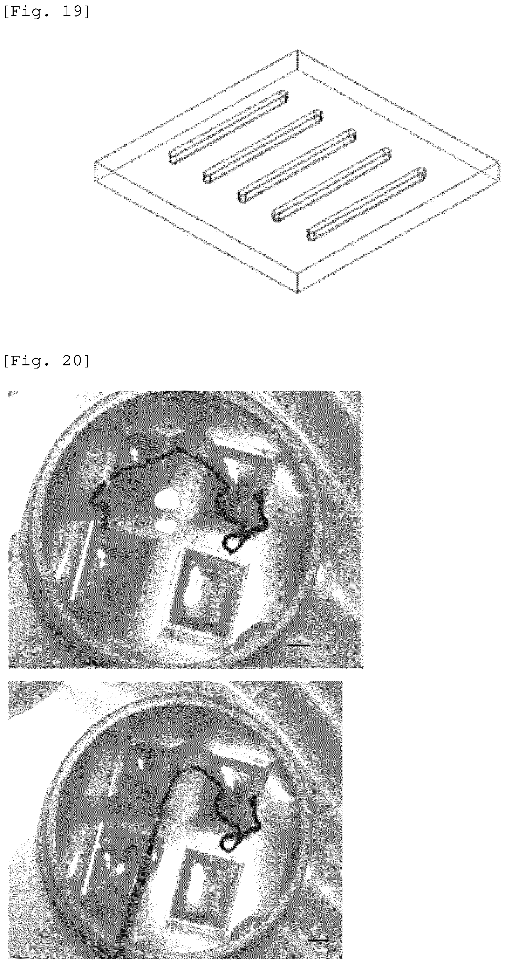

   Fig. 19 shows a COP groove structure.
[Fig. 20]
   Fig. 20 shows photographs of the moment when a string-like aggregate suspended in a medium made of a viscoelastic substance diluted 4-7 times with OptiMEM is suctioned with a cannula. Bar; 1 mm
[Fig. 21]

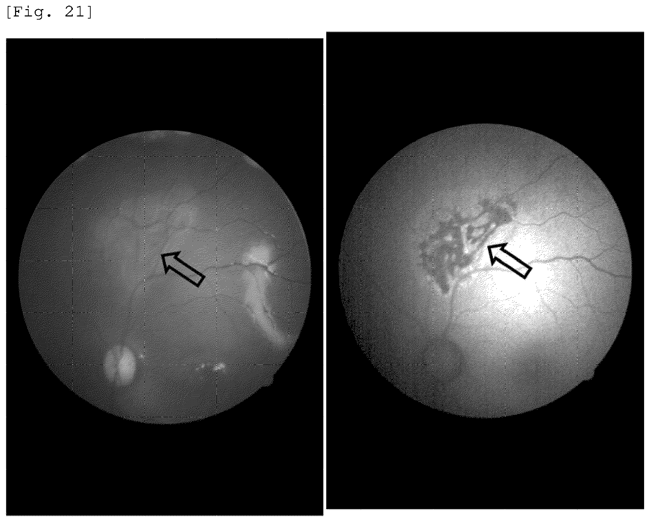

   Fig. 21 shows transplantation of string-like aggregates into monkey eyes.

### [Description of Embodiments]

### 1. String-like aggregates of retinal pigment epithelial cells

The present invention provides string-like aggregates of retinal pigment epithelial cells (hereinafter the string-like aggregates of the present invention).

In the present invention, the retinal pigment epithelial cell (hereinafter sometimes referred to as RPE cell) indicates an epithelial cell constituting the retinal pigment epithelium, and a progenitor cell thereof. Whether a retinal pigment epithelial cell or not can be confirmed by, for example, expression of cell markers (RPE65, CRALBP, MERTK, BEST1, etc.), cell forms (intracellular melanin dye deposition, polygonal and flat epithelium-like cell form, formation of polygonal actin bundle, etc.), and the like. The progenitor cell of retinal pigment epithelial cell means a cell directed to be induced to differentiate into retinal cell, and whether a progenitor cell or not can be confirmed by expression of cell markers (Mitf (pigment epithelial cell, pigment epithelial progenitor cell), Pax6 (pigment epithelial progenitor cell), Rx (retinal progenitor cell), OTX2 (retinal progenitor cell), RPE65 (pigment epithelial cell), BEST1 (pigment epithelial cell)), and the like. Functional evaluation of retinal pigment epithelial cell can be confirmed using, for example, secretability, phagocytosis capacity, and the like of cytokine (VEGF, PEDF, etc.) as an index. These functional evaluation and confirmation operations can be performed by those of ordinary skill in the art by setting appropriate conditions.

RPE cells can be obtained from any animal (e.g., human) that possesses RPE cells, or can be obtained by inducing differentiation from pluripotent stem cells by a method known per se. However, cells induced to differentiate from pluripotent stem cells are more preferred because they can be supplied in sufficient quantities or cells appropriate for the disease can be supplied. Pluripotent stem cells are not particularly limited as long as they have pluripotency enabling differentiation into all cells existing in the body, and also have the ability to proliferate. For example, embryonic stem cells (ES cells), embryonic stem cells derived from cloned embryos obtained by nuclear transplantation (ntES cells), spermatozoon stem cells (GS cells), embryonic germ cells (EG cells), induced pluripotent stem cells (iPS cells), pluripotent cells derived from cultured fibroblasts and myeloid stem cells (Muse cells), and the like are included. Preferred pluripotent stem cells are iPS cells. The origin of the pluripotent stem cells is not particularly limited, and for example, any animal in which the establishment of any of the following pluripotent stem cells has been reported, preferably mammals, more preferably humans, mice, rats, and the like, most preferably humans, can be mentioned.

iPS cells can be produced by introducing specific reprogramming factors into somatic cells in the form of DNA or proteins. They are artificial stem cells derived from somatic cells that have properties almost equivalent to ES cells, such as differentiation pluripotency and proliferation potency by self-replication (K. Takahashi and S. Yamanaka (2006), Cell, 126:663-676; K. Takahashi et al. (2007), Cell, 131:861-872; J. Yu et al. (2007), Science, 318:1917-1920; Nakagawa, M. et al., Nat. Biotechnol. 26:101-106 (2008); WO WO2007/069666).

The term somatic cell used in the present specification refers to any animal cells (preferably mammalian cells including humans) excluding germline cells such as ovum, oocyte, and the like and totipotent cells. Somatic cells include, without limitation, fetal somatic cells, neonatal somatic cells, and mature healthy or diseased somatic cells, and also include any of primary cultured cells, subcultured cells, and established cell lines. Specifically, somatic cells include, for example, (1) tissue stem cells (somatic stem cells) such as neural stem cell, hematopoietic stem cell, mesenchymal stem cell, pulp stem cell, and the like, (2) tissue progenitor cells, (3) differentiated cells such as lymphocyte, epithelial cell, endothelial cell, muscle cell, fibroblast (skin cell, etc.), hair cell, hepatocyte, gastric mucosa cell, enterocyte, splenocyte, pancreatic cell (pancreatic exocrine cell, etc.), brain cell, lung cell, kidney cell, adipocyte, and the like.

The reprogramming factor may be constituted of a gene specifically expressed in ES cells, a gene product or non-coding RNA thereof, or a gene that plays an important role in maintaining the undifferentiated state of ES cells, a gene product or non-coding RNA thereof, or a low molecular weight compound. Examples of genes included in the reprogramming factors include Oct3/4, Sox2, Sox1, Sox3, Sox15, Sox17, Klf4, Klf2, c-Myc, N-Myc, L-Myc, Nanog, Lin28, Fbx15, ERas, ECAT15-2, Tell, beta-catenin, Lin28b, Sall1, Sall4, Esrrb, Nr5a2, Tbx3, Glis1, and the like. These reprogramming factors may be used alone or in combination. Examples of the combination of the reprogramming factors include combinations described in WO2007/069666, WO2008/118820, WO2009/007852, WO2009/032194, WO2009/058413, WO2009/057831, WO2009/075119, WO2009/079007, WO2009/091659, WO2009/101084, WO2009/101407, WO2009/102983, WO2009/114949, WO2009/117439, WO2009/126250, WO2009/126251, WO2009/126655, WO2009/157593, WO2010/009015, WO2010/033906, WO2010/033920, WO2010/042800, WO2010/050626, WO2010/056831, WO2010/068955, WO2010/098419, WO2010/102267, WO2010/111409, WO2010/111422, WO2010/115050, WO2010/124290, WO2010/147395, WO2010/147612, Huangfu D, et al. (2008), Nat. Biotechnol., 26: 795-797, Shi Y, et al. (2008), Cellstem Cell, 2: 525-528, Eminli S, et al. (2008), Stem Cells. 26:2467-2474, Huangfu D, et al. (2008), Nat Biotechnol. 26:1269-1275, Shi Y, et al. (2008), Cellstem Cell, 3, 568-574, Zhao Y, et al. (2008), Cellstem Cell, 3:475-479, Marson A, (2008), Cellstem Cell, 3, 132-135, Feng B, et al. (2009), Nat Cell Biol. 11:197-203, R.L. Judson et al., (2009), Nat. Biotech., 27:459-461, Lyssiotis CA, et al. (2009), Proc Natl Acad Sci U S A. 106:8912-8917, Kim JB, et al. (2009), Nature. 461:649-643, Ichida JK, et al. (2009), Cellstem Cell. 5:491-503, Heng JC, et al. (2010), Cellstem Cell. 6:167-74, Han J, et al. (2010), Nature. 463:1096-100, Mali P, et al. (2010), Stem Cells. 28:713-720, Maekawa M, et al. (2011), Nature. 474:225-9.

RPE cells can be induced to differentiate from iPS cells (e.g., Neurosci. Lett., 458: 126-131, 2009; PLoS One, 8: 409-412, 2011). Alternatively, the methods described in WO2015/053375, WO2015/053376, WO2015/125941, WO2017/043605, and the like can also be used.

The string-like aggregate of the present invention is formed by adhesion between RPE cells. In this case, adhesion between cells refers to plane attachment between RPE cells in the string-like aggregate of the present invention. The plane attachment means that a cell attaches to another cell via planes. More particularly, the plane attachment between cells means that, for example, not less than 1%, preferably not less than 3%, more preferably not less than 5%, of the surface area of a cell adheres to the surface of another cell. A surface of a cell can be observed by staining with a reagent that stains membranes (e.g., DiI), immunostaining with cell adhesion factors (e.g., E-cadherin and N-cadherin).

### 2. Device for producing string-like aggregates of retinal pigment epithelial cells

The present invention also provides a device for producing string-like aggregates of retinal pigment epithelial cells (hereinafter the device of the present invention or said device).

As shown in Fig. 1, the device of the present invention has a base member 110 that is the main body of the device. The top surface 110a of the base member 110 is provided with one or more grooves 120. In Fig. 1, only one groove is depicted for illustration purposes. However, in a preferred embodiment, as will be described later, a plurality of grooves are arranged next to each other, and the cross sectional shape of the groove is V-shaped so that no top surface (flat plane) is present between the grooves. The groove 120 has a cavity part having a length corresponding to the length of the string-like aggregate to be formed and a width corresponding to the outer diameter of the string-like aggregate. In the example of Fig. 1, the bottom portion of the groove 120 is the cavity part. The cavity part is a concave mold in which retinal pigment epithelial cells seeded in the cavity part can grow into a string-like aggregate. With this configuration, as shown in Fig. 1(a), when an appropriate amount of retinal pigment epithelial cells a1 are seeded and cultured together with a liquid medium (not shown) in the groove 120 of said device, as shown in Fig. 1(b), a string-like aggregate A1 having a shape corresponding to the length and width of the cavity part is obtained. The string-like aggregate obtained by said device has a body surface (the lower surface of the string-like aggregate A1 in Fig. 1(b)) that is smoothly molded along the cavity part and a raised surface as a result of growing freely (the upper surface of the string-like aggregate A1 in Fig. 1 (b)).

### (Base member)

The overall shape and size of the base member are not particularly limited, and any shape or size is usable that has a top surface capable of forming one or more cavity parts capable of forming the string-like aggregate to be produced. A plate shape is exemplified as a preferred shape because it does not take up useless space, is easy to produce, and is easy to handle when producing the aggregate. In the example of Fig. 5(a), the overall shape of the base member 110 is a circle plate. When the base member is plate-shaped, the outer peripheral shape is not limited to a circular shape (that is, a disk shape), but may be a square, a rectangle, or the like. A disk shape is a preferable shape in that it can be placed in a commercially available petri dish, which is generally circular, without creating a dead space. Also, depending on the application, various incidental parts, such as a wall surrounding the outer periphery, a flange extending outward to the side, a handle for handling, a leg provided on the backside, typing or marking (concave or convex) and pasting of labels of a symbol specifying cavity (letter, number, QR code (registered trademark), and the like), typing or marking and pasting of labels of size scale to know the length of a cultured string-like aggregate, and the like, may be provided by integral molding or by attachment of separate parts.

### (Groove and cavity part)

As shown in Fig. 2 (a), the entire groove 120 may be the cavity part or, as shown in Figs. 2 (b), (c), (d), a structure in which the bottom of the groove 120 is the cavity part 121 and a top part 122 is set on the cavity part may be employed. In the embodiment shown in Fig. 2 (a), the string-like aggregate may protrude outward and rise from the groove opening.

As illustrated in Figs. 2 (b) to (d), when the internal structure of the groove 120 is a structure having a cavity part 121 as a bottom part and a top part 122 located above the cavity part 121, the cavity part 121 and the top part 122 may be integrally formed with each other as in the examples in these Figures, or as in the examples in Fig. 2 (e) and Fig. 3, may be separable into upper and lower parts (a combination of separate parts). The embodiment of Fig. 2(e) is a modified embodiment of Fig. 2(d). As in Figs. 2 (b), (c), and the like, the cavity part and the top part in various grooves may be separable. The boundary surface of separation does not necessarily have to be the same as the boundary surface between the cavity part and the top part, and may be a structure in which the cavity part and the top part are integrally formed, and the top part may be separated into two upper and lower parts. With such a structure that can be separated into two upper and lower parts, only the cavity part 121 can be exposed, as illustrated in Fig. 3(b), string-like aggregate A1 after culturing can be taken out more easily. In addition, a structure that can be separated into two upper and lower parts has production advantages in that there is no need to form a deep groove, and the degree of freedom in the groove profile is greater.

Fig. 3 shows specific examples of a device for making the cavity part and top part separable. In this embodiment, as shown in Fig. 3(a) as an assembled state, the base part 110 includes a first base part 111 and a second base part 112 disposed thereon. As shown in Fig. 3(b) as a separated state, the second base part 112 can be removed from the first base part 111. The first base part 111 is provided with the cavity part 121 of the groove 120, and the second base part 112 is provided with the top part 122 of the groove 120. Although not shown in the Figure, it is preferable that a positioning structure is provided to the first base part 111 and the second base part 112 so that they are connected to each other in a predetermined positional relationship (that is, a positional relationship in which one cavity part 121 and one top part 122 match to form one groove 120). Such positioning structure is not particularly limited and, for example, conventionally known positioning structures such as a positioning convex part, a positioning concave part that receives the convex part, and an outer peripheral shape that fits into each other without shifting (shape other than cylinder or round hole) can be appropriately adopted.

When the base part 110 can be separated into plural parts (e.g., the first base part 111 and the second base part 112) as mentioned above, the materials of the respective parts may be different from each other. For example, it is preferable to use, for the first base part 111 provided with the cavity, a material having biocompatibility (GCP: Good Clinical Practice compliant) capable of preferably growing string-like aggregates, and not allowing easy adhesion of cells so that string-like aggregates can be peeled off and removed easily from the cavity (such material may also be used for the second base part 112). Also, it is preferable to use materials that can be sterilized (e.g., materials that are heat resistant to autoclave heating, radiation resistant to gamma irradiation, compatible with EOG sterilization), and materials that are inexpensive and easy to process, for both the first base part 111 and the second base part 112.

### (Shape of string-like aggregate)

In order to explain the shape of the cavity part, the shape of the string-like aggregate is explained here.

In the present invention, the string-like aggregate is not particularly limited as long as it is an aggregate in which RPE cells are randomly aggregated into long and thin strings, it does not need to have clear apical and basal polarity like the retinal pigment epithelium in vivo, and has enough strength that the desired number of cells can be efficiently inserted and removed as a single unit by using a needle or tube-shaped transplant tip during transplantation. In addition, in the present invention, the "string-like" shape of a string-like aggregate is an elongated shape that clearly has a longitudinal direction, that is, a shape that extends long in one direction, compared to the shapes seen in known cell aggregates and cell sheets. As shown in Fig. 4(a) as a model for explanation, this elongated shape has a ratio (L1/d1) between the total length L1 and the outer diameter d1 of the body, when the string-like aggregate is made into a straight line, of 2 or more, more preferably not less than 5, further preferably not less than 10, more preferably not less than 20, particularly preferably not less than 50. If the shape has the above-mentioned ratio (L1/d1), especially the above-mentioned lower limit ratio of 2, the direction (that is, the longitudinal direction as a string-like aggregate) can be defined more than a simple cell aggregate. That is, in a tissue where orientation is important, a more desirable tissue can be obtained by appropriately defining the direction in advance.

In addition, if the string-like aggregate has the above-mentioned ratio, it can be transported as a single elongated structure by passing through the inside of the syringe needle, and the effect of being able to efficiently transport and place cells over a wide range in a single operation is more remarkably obtained. Moreover, it is possible to pass through narrow areas (e.g., minute through-holes, etc.) that are difficult to pass through for known cell sheets, and the effect of suppressing invasiveness into the living body can be more remarkably obtained.

When the ratio (L1/d1) falls below the aforementioned lower limit 2, the effect described above becomes less remarkable. The upper limit of the ratio (L1/d1) is not particularly limited. For example, even if the total length L1 is longer than the area of the target site where the string-like aggregate is to be transplanted, it may be cut as appropriate. From the viewpoints of avoiding excessively long groove of said device, the size of aggregates calculated from the number of cells to be transplanted, and limitations on transportation (e.g., length of syringe needle), the upper limit of the ratio (L1/d1) is exemplified to be about 1000, more preferably about 200, particularly preferably about 100.

The lower limit and upper limit of the aforementioned ratio (L1/d1) can be freely selected and combined in order to define the range of the ratio, for example, ratio (L1/d1)=2 - 1000, 5 - 1000, 10 - 1000, 20 - 1000, 50 - 1000, 10 - 200, 20 - 200, 50 - 200, 2 - 100, 5 - 100, 10 - 100, 20 - 100, or 50 - 100. The ratio (L1/d1) of the string-like aggregates obtained by the devices produced in the Examples of the present invention was about 2 to 100. The lower limit of the ratio is the minimum value necessary for the aggregate to be in a "string-like" form, but the upper limit of the ratio is not particularly limited. The upper limit of the ratio can be determined as appropriate depending on the space limitations of the culture facility, the range of the outer diameter d1, the labor required to divide into lengths suitable for cell transplantation, and the like.

### (Outer diameter d1 of body of string-like aggregate)

When a string-like aggregate is produced using said device, the cross sectional shape of the string-like aggregate (the shape of the cross section when the string-like aggregate is cut perpendicularly to its longitudinal direction) is, as illustrated in Figs. 2(a) to 2(d), that the lower part during culture has a shape that follows the cavity part, and the upper part has a shape after free proliferation. Therefore, the outer diameter d1 of the body of the string-like aggregate varies depending on the direction of measurement. For such outer diameter d1, an average value of outer diameter values measured in a plurality of directions may be adopted. However, as illustrated in Fig. 4(b), it is convenient to observe the string-like aggregates together with the device while they are grown in the cavity part (i.e., the inside of the groove is observed from above in Fig. 4(b)), and use the width of the body observed at that time as the outer diameter d1 of the string-like aggregate. Furthermore, in such observation, since the string-like aggregate becomes linear along the cavity part, the total length L1 can be easily measured.

### (Cross sectional shape of string-like aggregate)

The cross sectional shape of the string-like aggregate is not particularly limited and is generally amorphous. However, a shape close to a circular or elliptical shape is preferred because the distance from the tissue surface to the inside of the tissue is desirably constant from the aspects of supply of nutrients and oxygen and discharge of wastes, passage through narrow parts such as syringe needles and the like is more smooth, and a more smoothly curved cross sectional shape of the cavity part of the groove is more advantageous for cell proliferation and detachment.

### (Example of size of string-like aggregates when applied to living body)

As mentioned above, there is no limit on the length of the string-like aggregate, and when excessively long, it may be cut as appropriate when applied to a living body. Preferred sizes when applied to a living body include, for example, total length L1 of about 20 um - 200 mm, preferably about 20 µm - 40 mm, more preferably about 1 mm - 40 mm, further preferably about 5 mm - 40 mm. As one embodiment of the length compatible with the length of a 24 gauge needle, about 10 mm - 20 mm can be mentioned. These values of the total length L1 do not mean that there is only one most preferred range or value, but preferred ranges and values can be selected as appropriate depending on the size of the device, the acceptable length of groove, the number of cells required, the length of the conduit (e.g., injection needle) of the device for transplantation, and the like.

The outer diameter d1 is, for example, about 10 um (outer diameter corresponding to about one cell) - 300 µm (outer diameter corresponding to inner diameter of 24 gauge needle), preferably about 20 µm - 300 um, more preferably about 100 um - 250 um. These values of outer diameter d1 do not mean that there is only one most preferred range or value, but preferred ranges and values can be selected as appropriate depending on the size of the device, the acceptable inner diameter of the cavity of groove, the number of cells required, the inner diameter of the conduit (e.g., injection needle) of the device for transplantation, and the like.

When an injection needle is used to puncture the eyeball and the string-like aggregate is transplanted by sending same through the injection needle to a target site such as the fundus oculi region, the preferred inner diameter of the injection needle used is about 10 to 300 um. In this case, the outer diameter d1 of the string-like aggregate that can preferably move within the injection needle is, for example, about 80 to 95%, preferably about 90%, of the inner diameter of the injection needle. Therefore, in such a case, a preferred size range of the outer diameter d1 of the string-like aggregate is, for example, about 100 µm to 270 µm. In this case, a preferred length L1 of the string-like aggregate is, for example, about 20 µm - 200 mm, preferably about 20 µm - 40 mm, more preferably, about 1 mm - 40 mm, further preferably, about 5 mm - 40 mm, because it is effective for transplantation and is compatible with the length of the injection needle. One embodiment of the length compatible with the length of a 24 gauge needle is about 10 mm - 20 mm. Therefore, the preferable ratio (L1/d1) when sending the string-like aggregate using an injection needle is, for example, 40 to 200, preferably about 80. Further, the number of cells at that time is, for example, about 1.5×10⁵ - 4.5×10⁵.

As described above, the string-like aggregate of the present invention can be characterized by the aforementioned ratio (L1/d1) between the total length L1 and the outer diameter d1 of the body. Therefore, the string-like aggregate of the present invention may be characterized in that the ratio (L1/d1) between the total length L1 and the outer diameter d1 of the body is 2 to 1,000. The L1/d1 may be generally 2 - 1,000, and may also be 5 - 1,000, 10 - 1,000, 20 - 1,000, 50 - 1,000, 10 - 200, 20 - 200, 50 - 200, 2 - 100, 5 - 100, 10 - 100, 20 - 100, or 50 - 100.

The string-like aggregates of the present invention can be further characterized by the cross sectional shape thereof. As mentioned above, the cross sectional shape of the string-like aggregate is not particularly limited and is usually amorphous, but a shape close to a circular or elliptical shape is preferred. Therefore, the string-like aggregate of the present invention may have further characteristic that the cross sectional shape is a circular or elliptical shape.

### (Cross sectional shape of cavity part)

The cavity part within the groove of said device preferably has a cross-sectional shape and length corresponding to the shape of the string-like aggregate described above. The cross sectional shape of the cavity part is the shape of the cross section when the cavity part is cut perpendicularly to the longitudinal direction of the groove. The cross sectional shape of the cavity part can also be appropriately determined by further considering cell culture property, mold releasability, and the like. Preferred cross sectional shape includes, for example, arc, semicircle, rectangle with an open top, U-shape, and V-shape. The aforementioned bent part (corner) of the rectangle or V-shape is preferably rounded because string-like aggregates are easily removed using tweezers, syringe needles, and the like, and the cross sectional shape of the string-like aggregate is preferably close to a circle. The radius of curvature of the roundness of the corner is not particularly limited, but a preferred value is about half the outer diameter d1 of the string-like aggregate (e.g., radius of about 5 µm to 150 µm). The width W1 of the cavity part shown in Fig. 4(b) is preferably the same as the outer diameter d1 of the string-like aggregate.

### (Preferred embodiment of groove)

In a preferred embodiment, as shown in Figs. 2(c), (d), Fig. 4(b), the groove has a cavity part 121 at its bottom and a top part 122 above the cavity part, and the cross sectional shape of the top part 122 is such that the width increases from the bottom side toward the opening side. The cross sectional shape of the entire groove is V-shaped, and the deepest part thereof is rounded as the above-mentioned cavity part. Due to this V-shaped cross sectional shape, cells to be seeded in suspension are guided into the bottom cavity part, seeding operation becomes easy, the string-like aggregates come out easily from within the groove, facilitating the operation for taking out.

The depth of the entire groove (B1 in Fig. 4 (b)) is not particularly limited, and is preferably about 5 - 5000 µm (about 5 mm shorter than the depth of general petri dish), more preferably about 5 - 1600 µm.

The depth of the cavity part (size b1 shown in Fig. 4(b)) is preferably about 5 to 300 µm, more preferably about 5 to 250 um, depending on the outer diameter d1 of the string-like aggregate to be formed.

The width of the opening of the groove (W2 shown in Fig. 4(b)) is not particularly limited and is preferably about 560 µm (outer diameter of 24 gauge needle) - 5000 um (tip diameter of 1 mL pipette chip), more preferably about 1000 µm - 3000 µm (approximate value of the device), more preferably about 1500 µm - 2500 um.

When the cross sectional shape of the entire groove is V-shaped as described above, the angle of inclination of the inner wall (01 shown in Fig. 4(b)) is preferably about 1 to 90 degrees, and more preferably about 40 to 70 degrees. The cross sectional shape of the entire groove can be appropriately determined depending on the amount of cell suspension required for culture, the number of cells, the size of the container to be stored, and the like.

The length of the groove is preferably the same as the length of the string-like aggregate to be formed, but may be longer.

The top part of the cross sectional shape of the groove may be a straight line as shown in Fig. 4 (b), or as shown in Fig. 5 (b), may be a curved line that curves so that the width increases from the bottom side toward the opening side. Such a curvature can also be interpreted as a rounded edge of the straight V-shaped opening part as shown in Fig. 4(b).

### (Groove arrangement pattern)

In a preferred embodiment, as shown in Fig. 5, the top surface 110a of the base member 110 is provided with a plurality of (five in the example of the Figure) grooves 120 in parallel stripes. As a result, a plurality of string-like aggregates can be obtained in one culture, thereby improving production efficiency. The number of grooves is not particularly limited, and may be appropriately determined along with the size of the base member depending on the scale of production, from experimental use to commercial use. In the example shown in Figs. 5(a), (b), the top part in the cross sectional shape of groove 120 is a curved line that becomes wider as it goes from the bottom side toward the opening side, and is smoothly connected to the top surface of the base member and the adjacent opening. In Fig. 5 (b), the boundary line between adjacent grooves and the boundary line 130 between the groove and the top surface 110a of the base member 110 are shown by dashed lines for easy understanding.

When arranging multiple grooves in parallel stripes, in a preferred embodiment, as shown in Fig. 5, the wall part separating adjacent grooves from each other does not have a flat plane part on the top surface of the base member, and the openings of adjacent grooves contact each other. Such constitution is preferably achieved when the cross sectional shape of the entire groove is V-shaped. Such constitution allows the grooves to be arranged more densely. When culture is performed using said device, culture may be performed by injecting the culture medium and cells only into the groove, or said entire device may be immersed in the culture medium and cells may be seeded and cultured. It is preferable that said entire device is immersed in a culture medium and then cells are seeded, because the seeded cells are less likely to be deposited on the flat plane between the grooves and more cells fall into the grooves.

### (Material of base member)

The material of the base member (i.e., material of said device) is not particularly limited, and materials suitable for cell culture, such as metals and polymers, that are inexpensive and easy to produce are preferred. Glass and various polymer materials (e.g., polystyrene, polycarbonate, acrylic, silicone, cyclo olefin polymer (COP), etc.), which have been conventionally used as materials for containers for cell culture, are preferred. Among these, polydimethylsiloxane (PDMS), which is one type of silicone, has advantages such as being easy for forming using a mold, capable of being heated in an autoclave, being flexible, having high chemical resistance, and being applicable to microfabrication processes. Therefore, it is exemplified as a more preferred material.

### (Production method of said device)

The production method of said device may be determined as appropriate depending on the material. Various methods can be used, including plastic deformation using a press, resin forming using a mold, cutting, 3D printing, subtractive groove formation on the top surface of the base member, additive groove processing on the top surface of the base member, laser processing, and the like.

When the material of the base member is PDMS, a production method including using a forming mold having a convex mold corresponding to the shape of the groove, pouring fluid PDMS before curing into the forming mold (or bring it into contact with the convex mold), and curing the PDMS in that state to obtain a molded product (said device) is preferred. PDMS is cured and becomes PDMS by heating at, for example, 80°C for about 3 hr.

### (Preferred production method of forming mold)

One embodiment of a preferred production method of a forming mold is shown. Fig. 6 shows one embodiment of a preferred production method of a forming mold.

In the example of the production method, first, as shown in Figs. 6(a), (b), a protrusion 220 serving as a convex core corresponding to the shape of the groove is formed on one surface 210a of a mold substrate 210 in accordance with the center-to-center pitch of the groove. Then, as shown in Figs. 6(c), (d), a fluid polymer raw material (e.g., PDMS) before curing is applied so as to cover the protrusion 220 and cured to obtain a V-shaped convex mold 240. Here, when applying the polymer raw material, the cross sectional shape of the coating layer 230 is shaped into a V-shaped convex mold corresponding to the shape of the groove to be molded, by utilizing the fluidity, viscosity, surface tension, etc. of the polymer raw material, as shown in Fig. 6(c). As a result, a V-shaped convex mold can be easily obtained from a core having a simple shape.

The conditions for controlling the liquid polymer raw material into a V-shape as shown in Fig. 6(c) include, for example, conditions under which PDMS before curing and having a viscosity of about 3.5 (Pa·s) at 25 degrees is applied to cover the aforementioned protrusion, which is left standing while being defoamed in a vacuum desiccator at room temperature (about 23 to 27°C) for 30 min, and then cured at 80°C for 3 hr.

In the Example of the present invention, the parts shown in Figs. 6(a), (b) (mold substrate 210 provided with protrusion 220) were produced using a 3D printer (the material was polylactic acid (PLA resin)). The forming mold 200 having a V-shaped convex mold 240 as shown in Fig. 6(c) may be produced using a 3D printer, and the surface may be further finished.

Fig. 7 is a cross sectional view illustrating how said device is molded using the forming mold described above. As shown in Fig. 7(a), a forming mold 200 is placed on the bottom of a molding container (not shown), or a molding container is formed by surrounding the forming mold 200 with a wall member, a fluid polymer raw material before curing is poured thereinto, and the polymer raw material in that state is cured to obtain a molded product (said device) 100. Then, as shown in Fig. 7(b), the molded product formed from the forming mold 200 is detached to obtain said device 100. Fig. 8(a) is a photographic diagram showing the substrate surface of a mold produced in the Example of the present invention, in which a protrusion 220 is provided on one main surface of the mold substrate 210. Fig. 8(b) is a photographic diagram showing the substrate surface of a forming mold produced using the forming mold of Fig. 8(a) in the Example of the present invention, in which a protrusion 220 is provided on one main surface of the mold substrate 210.

### 3. Production method of string-like aggregates of retinal pigment epithelial cells

The present invention also provides a method for producing retinal pigment epithelial cells aggregated into strings (hereinafter the production method of the present invention). The production method of the present invention includes the following steps.
(1) As illustrated in Fig. 9(a), a step of seeding retinal pigment epithelial cells a1 suspended in a liquid medium in the groove 120 in the device 100 of the present invention.
(2) As illustrated in Fig. 9(a), a step of culturing retinal pigment epithelial cells a1 to form string-like aggregates A1 of retinal pigment epithelial cells (the Figure shows one string-like aggregate being removed from a groove).

In step (1), retinal pigment epithelial cells suspended in a medium are seeded in the groove of the device of the present invention.

As a liquid medium in which retinal pigment epithelial cells are suspended in step (1), a medium conventionally used for culturing animal cells can be prepared as a basal medium. Examples of the basal medium include media that can be used for culturing animal cell such as BME medium, BGJb medium, CMRL 1066 medium, Glasgow MEM (GMEM) medium, Improved MEM Zinc Option medium, IMDM medium, Medium 199 medium, Eagle MEM medium, αMEM medium, DMEM medium, F-12 medium, DMEM/F12 medium, IMDM/F12 medium, ham medium, RPMI1640 medium, Fischer's medium, or a mix medium of these and the like. The medium used for culture may be a serum-containing medium or a serum-free medium.

In the present specification, the "serum-containing medium" means a medium containing unadjusted or unpurified serum. As the serum concentration of the serum-containing medium, a medium generally containing unadjusted or unpurified serum at not more than 5%, preferably not more than 3%, can be mentioned. The medium may contain a fatty acid, lipid, amino acid (e.g., non-essential amino acids), vitamin, growth factor, cytokine, antioxidant, 2-mercaptoethanol, 1-monothioglycerol, pyruvic acid, buffering agent, inorganic salts, and so on.

In the present specification, the "serum-free medium" means a medium not containing an unadjusted or unpurified serum. In the present invention, a medium containing purified blood-derived components and animal tissue-derived components (e.g., growth factor) is also included in the serum-free medium unless unadjusted or unpurified serum is contained therein.

The serum-free medium may contain a serum replacement. Examples of the serum replacement include one appropriately containing albumin, transferrin, fatty acid, collagen precursor, trace element, 2-mercaptoethanol or 3' thiolglycerol, or equivalents of these, and so on. Such serum replacement may be prepared by, for example, the method described in WO 98/30679. The serum replacement may be a commercially available product. Examples of such commercially available serum replacement include Knockout (trademark) Serum Replacement (Life Technologies; now ThermoFisher: hereinafter sometimes to be indicated as KSR), Chemically-defined Lipid concentrated (manufactured by Life Technologies) and Glutamax (trademark) (manufactured by Life Technologies), B27 (manufactured by Life Technologies), N2 supplement (manufactured by Life Technologies), and ITS supplement (manufactured by Life Technologies).

The serum-free medium used for culture may appropriately contain a fatty acid or lipid, amino acid (e.g., non-essential amino acids), vitamin, growth factor, cytokine, antioxidant, 2-mercaptoethanol, pyruvic acid, buffering agent, inorganic salts, and so on.

The medium in which retinal pigment epithelial cells are suspended in step (1) may further contain a ROCK inhibitor.

In the present invention, the ROCK inhibitor is not limited as long as it is a substance that inhibits the action of Rho kinase (ROCK). Rho kinase (ROCK) was discovered as a serine/threonine kinase located downstream of the low molecular weight G protein Rho. The Rho/ROCK signal transduction pathway is involved in various cell functions such as actin cytoskeleton and cell adhesion. In passage culture of iPS cells, it is necessary to disperse the cells. However, it is known that when these stem cells are cultured in a dispersed state, cell death occurs due to apoptosis. Apoptosis caused by dispersion involves the Rho/ROCK signal transduction pathway, and it has been reported that addition of a ROCK inhibitor suppresses apoptosis (apoptosis suppressive action). Furthermore, it has been reported that addition of a ROCK inhibitor during cryopreservation of cells increases the cell survival rate after thawing (cell survival rate improving effect). Based on these reports, ROCK inhibitors have been added during proliferation culture of iPS cells. On the other hand, regenerative medical products generally use basal media such as equilibrium saline solutions and DMEM/F12 medium, and do not contain exogenous components. In addition to the above-mentioned actions, the present inventors have found that when string-like aggregates are produced using an appropriate concentration of ROCK inhibitor, as shown in the Examples described below, the string-like aggregates become more stable, and further a new action of promoting the engraftment and cell growth of string-like aggregates of RPE cells at the site of transplantation is provided.

In the present invention, the ROCK inhibitor may be any molecule as long as it has the same or substantially the same actions as the above-mentioned actions found newly (string-like aggregate formation promoting action). The "substantially the same" indicates that the actions thereof are qualitatively (e.g., physiologically or pharmacologically) the same. Therefore, even though the aforementioned actions are preferably equivalent, the level of those actions (e.g., about 0.1- to about 10-fold, preferably about 0.5- to about 2-fold) may be different. The aforementioned actions can be measured according to a method known per se. Examples of such ROCK inhibitor include Y-27632 dihydrochloride, Y-27632, Fasudil Hydrochloride, Chroman 1, SLx-2119, HSD1590, GSK269962A hydrochloride, Exoenzyme C3, clostridium botulinum, Ripasudil, Afuresertib, Thiazovivin, GSK269962A, RKI-1447, Y-33075, GSK429286A, AT13148, H-1152 dihydrochloride, Y-33075 dihydrochloride, LX7101, SAR407899, ROCK-IN-2, Afuresertib hydrochloride, Hydroxyfasudil, GSK180736A, BDP5290, SR-3677, CCG-222740, CMPD101, Rho-Kinase-IN-1, SAR407899 hydrochloride, ROCK inhibitor-2, ZINC00881524, H-1152, Hydroxyfasudil hydrochloride, Fasudil, ROCK2-IN-2, Verosudil, SB-772077B dihydrochloride, GSK-25, CRT0066854 hydrochloride, Ripasudil free base, ROCK-IN-1, and the like, with preference given to Y-27632 dihydrochloride and Y-27632.

ROCK inhibitor can be produced by a method known per se. Furthermore, a commercially available ROCK inhibitor can also be purchased and used. For example, Y-27632 can be purchased from FUJIFILM Wako Pure Chemical Corporation and the like. In addition, Ripasudil is commercially available under the trade name of Granatec (registered trademark) (Kowa), and Fasudil Hydrochloride is commercially available under the trade name of Eryl (registered trademark) (Asahi Kasei Pharma) and the like.

The concentration of the ROCK inhibitor contained in the medium is not particularly limited as long as it allows RPE cells to form string-like aggregates in the groove of the device of the present invention. It is generally 0 µM - 20 µM, preferably 2 µM - 10 µM. When the concentration of the ROCK inhibitor is too high, there is a concern that the aggregates of the present invention may aggregate too tightly, which may affect the spread of cells after transplantation.

In step (2), the retinal pigment epithelial cells seeded in step (1) are cultured.

In step (2), the density of the retinal pigment epithelial cells cultured in the groove is not particularly limited as long as the retinal pigment epithelial cells form string-like aggregates in the groove of the device of the present invention. It is generally not less than 2.5×10³ cells/mL, preferably 2.5×10³ cells/mL - 5×10⁵ cells/mL, more preferably 1×10⁵ cells/mL - 2×10⁵ cells/mL. When the density of cells in the groove is higher than these, there occur concerns that the outer diameter of the body of the string-like aggregate to be formed may become larger and the cells after transplantation may not spread, and the transplanted cells may form a multilayer at the site of operation.

The culture period of the retinal pigment epithelial cells in step (2) is not particularly limited as long as string-like aggregates of retinal pigment epithelial cells can be obtained in step (2). It is generally 1 day to 30 days, preferably 2 days to 7 days. When the culture period is longer or shorter than these, there is a concern that the aggregates of the present invention may be released from the device of the present invention when the medium is replaced.

The culture conditions such as culture temperature and CO₂ concentration in step (2) can be appropriately determined. The culture temperature is, for example, about 30°C to about 40°C, preferably about 37°C. The CO₂ concentration is, for example, about 1% to about 10%, preferably about 5%.

### 4. Therapeutic drug for diseases caused by disorder of retinal tissues

The string-like aggregate of retinal pigment epithelial cells of the present invention obtained as described above is useful for transplantation therapy of diseases caused by disorders of retinal tissues, for example, diseases caused by disorders of retinal pigment epithelium. Therefore, the present invention provides a therapeutic drug for a disease caused by a disorder of a retinal pigment epithelium, containing a string-like aggregate of retinal pigment epithelial cells (the therapeutic drug of the present invention).

The therapeutic drug of the present invention contains an effective amount of a string-like aggregate of retinal pigment epithelial cells and a pharmaceutically acceptable carrier. The string-like aggregates of retinal pigment epithelial cells contained in the therapeutic drug of the present invention include those produced by the production method of the present invention.

As a pharmaceutically acceptable carrier, a physiological aqueous solvent (saline, buffer, serum-free medium, etc.) can be used. Where necessary, in a transplantation therapy, a medicament containing a tissue or cells to be transplanted may contain conventionally used preservative, stabilizer, reducing agent, isotonizing agent, and the like.

The therapeutic drug of the present invention can be produced as a suspension by suspending the string-like aggregates of retinal pigment epithelial cells in an appropriate physiological aqueous solvent. Where necessary, it may be cryopreserved by adding a cryopreservative, and when in use, thawed and washed with buffer for use in a transplantation therapy.

The string-like aggregate of the retinal pigment epithelial cells of the present invention can be used as a therapeutic drug for a disease caused by a disorder of a retinal pigment epithelium or to supplement the corresponding atrophy/damaged site in the atrophy or damaged state of retinal pigment epithelium. A disease due to a disorder of retinal pigment epithelium, and atrophy and damaged state of retinal pigment epithelium can be treated by transplanting a retinal pigment epithelial cells string-like aggregate of the present invention to a patient with a disease due to a disorder of retinal pigment epithelium, or atrophy and damaged state of retinal pigment epithelium, who requires transplantation, to supplement the retinal pigment epithelium. Examples of the disease due to a disorder of retinal pigment epithelium, and disease with atrophy and damaged state of retinal pigment epithelium include ophthalmologic diseases such as age-related macular degeneration, retinitis pigmentosa, and retinal pigment epithelial hiatus.

The content of string-like aggregates of retinal pigment epithelial cells in the therapeutic drug of the present invention is not particularly limited as long as string-like aggregates of retinal pigment epithelial cells containing a therapeutically effective amount of RPE cells are contained in a suspension (e.g., 50 - 500 µL, preferably 100 - 300 µL) to be injected into the disease site, namely, a retinal pigment epithelium-defective site in macular degeneration or retinitis pigmentosa. For example, a suspension of string-like aggregates of retinal pigment epithelial cells can be injected such that the RPE cells are 100 - 20,000 cells/µL, preferably 1,000 - 10,000 cells/µL. The therapeutic drug of the present invention does not have difficulty in controlling cells at the transplant site, such as in a suspension of RPE cells, and the transplanted cells also do not form an epiretinal membrane (ERM) within the vitreous. Furthermore, unlike RPE sheets, the therapeutic drug of the present invention does not require time or cost to prepare sheets, and does not require invasive surgical procedures involving large incisions.

The therapeutic drug of the present invention can be transplanted by injecting same using a transplantation device including a suitable syringe and a needle (e.g., MedOne0 (registered trademark) Poly Tip (registered trademark) Cannula 25 g/31 g, etc.) into, for example, under the retina of a mammal (e.g., human, mouse, rat, etc., preferably human) having a retinal disease such as macular degeneration (e.g., age-related macular degeneration, retinitis pigmentosa, etc.) or retinitis pigmentosa. As a medium to be used when transplanting the therapeutic drug of the present invention, a medium obtained by diluting a viscoelastic substance (shell gun, viscoat, etc.) 4 to 8 times with OptiMEM can be used. By using the medium for transplantation, the string-like aggregates of retinal pigment epithelial cells of the present invention can be smoothly aspirated and discharged.

While the present invention is explained in detail by referring to the following Examples, the present invention is not limited thereto.

### [Example]

### Experimental procedure

All animal experimental protocols were approved by the Animal Care Committee of the RIKEN Center for Biosystems Dynamics Research (BDR) and were conducted in accordance with local guidelines and the ARVO Statement for the use of animals in eye and vision research. hiPS cells were generated with the approval of the Ethics Committee of the RIKEN Center for Biosystems Dynamics Research (BDR) and with informed consent from volunteers.

### Example 1

### Production of PDMS based-culture device for production of string-like aggregates of hiPSC-RPE

A culture device for PDMS base having a groove of length 19.5 mm, width 1 mm, depth 1.6 mm was produced. The production process of this device is summarized. First, a mold for this device was created by 3D printing using polylactic acid (PLA) filament. After cutting the 3D printed mold and applying PDMS to adjust the shape of groove, a mold release agent (Novec1720, manufactured by Sumitomo 3M) was applied onto the mold. PDMS and a curing agent for PDMS (Cypot 184W/C) (manufactured by Dow Corning Toray) were mixed at a ratio of 10:1, poured onto the 3D printed mold, and deaerated for about 1 hr. The 3D printed mold and PDMS mixture were then placed in an oven at 80°C for 3 hours, whereby cured PDMS was collected from the 3D printed mold and trimmed. The radius of curvature of the groove bottom was measured using a confocal scanning laser microscope (KEYENCE, VK-8710) and was found to be R 0.2 mm (Fig. 10).

### Preparation of human iPSC (hiPSC) and hiPSC-derived RPE cell (hiPSC-RPE)

By introducing six reprogramming factors (OCT3/4, SOX2, KLF4, L-MYC, LIN28, and p53 carboxy-terminal dominant negative fragment) into peripheral blood mononuclear cells (PBMCs) of healthy volunteers, M8 human iPSC line was established in the laboratory of the present inventors. Briefly, episomal vectors (pCE-hOCT3/4, pCE-hSK, pCE-hUL, pCE-mp53DD, pCXB-EBNA1 purchased from Addgene) were electroporated into PBMC and then iPSC-like colonies were excised as previously described (Okita et al., 2011). After several passages, the present inventors performed PCR analysis using DNA extracted from the iPSC-like cells and confirmed that no residual episomal vector could be detected using plasmid-specific primers.

The 201B7 line and 253G1 hiPSC line (Nakagawa et al., 2008; Takahashi et al., 2007) were obtained from the RIKEN BioResource Center. In order to visualize the G1 phase of the cell cycle and the entire cell body, a modified Fucci 201B7 line (201B7modFucci) was produced in the laboratory of the present inventors. Briefly, mCherry-hCdt1 (30/120)-P2A-mVenus region was excised from tFucci(CA)2/pCSII-EF vector (gift from Dr. A. Miyawaki; https://cfm.brc.riken.jp/lentiviral-vectors/plasmid-list/) and ligated into pAAVS1-Nst-CAG-DEST vector obtained from Addgene (Plasmid #80489). 201B7 was cotransfected with a modified Fucci construct encoding a gRNA designed to target the AAVS1 locus and a Cas9 vector (Addgene Plasmid #62988) using DNA-in CRISPR delivery medium (MTI-GlobalStem, Gaithersburg, MD) (Oceguera-Yanez et al., 2016). After transfection, mVenus-positive 201B7 colonies were dissociated, passaged, and colonies derived from a single clone were picked.

hiPSC-RPE cells were differentiated using the SFEBq method (Kuwahara et al., 2015, 2019; Nakano et al., 2012). Briefly, 5 µM SB431542 (Sigma-Aldrich) and 300 nM SAG (Enzo Life Sciences, Inc) were added to iPS cells the day before differentiation induction. On day 0, iPS cells were suspended in growth factor-free chemically defined medium (gfCDM) supplemented with IMDM:F12 (1:1, Life Technologies), 10% Knockout Serum Replacement (KSR), 1% Chemically Defined Lipid Concentrate (Life Technologies), 30 w/v% BSA (fatty acid free), 450 µM monothioglycerol, 30 nM SAG, 20 µM Y-27632 (Fujifilm Wako Pure Chemical Industries, Ltd.), and penicillin-streptomycin and cultured in PrimeSurface 96V (Sumitomo Bakelite Co., Ltd.). 1.5 nM BMP4 was added on day 6, and half of the gfCDM was replaced every 3 days. On day 18, using DMEM/F12-Glutamax medium containing 1% N2 supplement (Life Technologies), 3 µM CHIR99021 (Stemgent, Cambridge), and 5 µM SU5402, the cells were transferred into Ultra Low Culture Dish (Corning Incorporated) (Corning Incorporated). From day 22 onwards, the cells were cultured in DMEM/F12-Glutamax medium (Life Technologies) containing 1% N2 supplement, 10% fetal bovine serum (Biosera), 0.5 µM retinoic acid (sigma), 0.1 mM taurine (sigma), and 1x antibiotic antimycotic (gibco).

Between days 30 and 60, pigmented colonies were picked up and plated in a 12-well plate coated with iMatrx511 in a 1:1 mix medium of "RPE adhesion medium" and "RPE maintenance medium" (hereinafter referred to as "mix medium"). The "RPE adhesion medium" contains DMEM/F-12 (Sigma-Aldrich), 10% fetal bovine serum (SAFC Biosciences Inc.), gentamicin solution (Sigma-Aldrich), and the "RPE maintenance medium" contains DMEM-low Glucose (Sigma-Aldrich), 30% F-12 (Sigma-Aldrich), 2% L-glutamine solution (Sigma-Aldrich), 2% B-27TM supplement (50X) (Thermo Fisher Scientific Inc.), and gentamicin solution (Sigma-Aldrich). Once the cells attached, the medium was changed to RPE maintenance medium containing 10 ng/mL basic fibroblast growth factor (bFGF). After one passage, the cell culture was expanded into RPE maintenance medium, and the cells were stocked at -150°C using a STEM-CELL BANKER (Nippon Zenyaku Kogyo Co., Ltd.).

For each experiment, stock cells were thawed and seeded in the mix medium, then the medium was changed to RPE maintenance medium supplemented with 10 ng/mL bFGF and 0.5 µM SB431542, and the medium was changed every several days until the cells were used. RPE cells differentiated from the 253G1 line were prepared as described in Kitahata et al., 2019.

### Production of string-like aggregate

After thawing, hiPSC-RPE cells were cultured for 2 weeks, the cells were collected, and seeded in the number described in each experiment into each groove of the PDMS-based culture device, using 15 µl RPE maintenance medium containing the optimal concentration of Y-27632 (concentrations indicated for each experiment).

### Analysis of expanded area of RPE from plated string-like aggregates

The string-like aggregates were released from the grooves of the device, and the string-like aggregates were seeded onto an uncoated 24-well plate (Corning Incorporated) containing the mix medium. After adhesion of the string-like aggregates (2 to 3 days later), the medium was replaced with RPE maintenance medium (containing bFGF and SB431542). The dishes under culture were photographed using the IncuCyte Zoom system (Essen BioScience). From the obtained image data, expanded cells were manually marked using Fiji, and the expanded area was measured.

### Immunohistochemistry

The cultured string-like aggregates or cells were fixed with 4% paraformaldehyde for 15 min at room temperature. The transplanted eye was fixed with 4% paraformaldehyde at 4°C for 1 hr, and the cornea was removed after fixation. Samples for cryosectioning were immersed in 30% sucrose for at least one day and frozen in OCT compound (Sakura Finetek Japan Co., Ltd.) to prepare cryosections with a thickness of 10 µm. Samples were permeabilized with 0.2% Triton X-100 in phosphate buffered saline for 30 min, blocked with Blocking One (Nacalai Tesque) at room temperature for 1 hr, and incubated overnight at 4°C with primary antibody diluted with antibody diluent (Dako). The antibodies used are listed in Table 1. The secondary antibody was treated for 1 hr at room temperature, and images were obtained using a confocal microscope (LSM700; Carl Zeiss) and a fluorescence microscope (BZ-X810; KEYENCE).

**[Table 1]**

| Antigen | Host | Company | Product | Dilution |
|---|---|---|---|---|
| collagen type IV | Mouse | abcam | ab6311 | 1:400 |
| Ezrin | Rabbit | R&D Systems | MAB7239 | 1:1000 |
| Z01 | Mouse | Invitrogen | 33-9100 | 1:1000 |
| Laminin | Rabbit | abcam | ab11575 | 1:400 |
| MiTF | Mouse | abcam | ab80651 | 1:500 |
| Ku80/XRCC5 (Hunu) | Goat | R&D Systems | AF5619 | 1:200 |

### PCR of RPE marker gene

Total RNA was extracted from cells using the RNeasy Micro Kit (Qiagen), and cDNA was synthesized using the SuperScript III Reverse Transcriptase Kit (Invitrogen). Table 2 shows the sequences of the RPE markers (BEST1, RPE65, CRALBP) and housekeeping gene (GAPDH) primers. The PCR reaction was performed using Blend Taq -Plus- (TOYOBO Co., Ltd.). Thermal cycle conditions were as follows. One cycle at 94°C for 180 seconds, 32 cycles of denaturation at 94°C for 30 seconds, priming at 58°C for 30 seconds, and extension at 72°C for 60 seconds was performed, followed by one cycle at 72°C for 60 seconds.

**[Table 2]**

| Gene | | Sequence (5' →3' ) |
|---|---|---|
| BEST1 | F | 5' -TAGAACCATCAGCGCCGTC-3' **(SEQ ID NO:1)** |
| | R | 5' -TGAGTGTAGTGTGTATGTTGG-3' **(SEQ ID NO:2)** |
| RPE65 | F | 5' -TCCCCAATACAACTGCOACT-3' **(SEQ ID NO:3)** |
| | R | 5' -CCTTGGCATTCAGAATCAGG-3' **(SEQ ID NO:4)** |
| CRALBP | F | 5' -GAGGGTGCAAGAGAAGGACA-3' **(SEQ ID NO:5)** |
| | R | 5' -TGCAGAAGCCATTGATTTGA-3' **(SEQ ID NO:6)** |
| MERTK | F | 5' -TCCTTGGCCATCAGAAAAAG-3' **(SEQ ID NO:7)** |
| | R | 5' -CATTTGGGTGGCTGAAGTCT-3' **(SEQ ID NO:8)** |
| GAPDH | F | 5' -ACCACAGTCCATGCCATCAC-3' **(SEQ ID NO:9)** |
| | R | 5' -TCCACCACCCTGTTGCTGTA-3' **(SEQ ID NO:10)** |

### In vitro RPE injury model

RPE cells cultured at confluence in a 24-well plate were treated with 6 µg/mL MitomycinC (Kyowa Hakko KIRIN), and the next day, a portion of the RPE cells were removed by scraping using a cell scraper with a width of about 6 mm. Cell suspensions (2×10⁵) or string-like aggregates were plated ("grafted") onto or into the scratched area of RPE. Cells were maintained in 0.5 ml of medium and incubated for 24 hours before medium exchange, and the cells were harvested for ELISA at the time of each intervention. The schedule of the experimental design and timing of medium collection is shown in Fig. 11A.

### ELISA for VEGF and PEDF

ELISA for VEGF and PEDF was performed according to the manufacturer's protocol for the VEGF Human ELISA Kit (Life Technologies) and Human PEDF ELISA Kit (Biovendor), respectively.

### Transplantation into rat eyes

Immunodeficient F344/NJc1-rnu/rnu female nude rats (6 weeks old) were obtained from CLEA Japan. A disposable micropipette (Drummond, 1-000-0500) was pulled with a micropipette puller (Sutter, P-97/IVF Puller), and the tip was cut and sharpened using a microgrinder (Narishige, EG-400). The micropipette was then attached to an electrode holder (WPI, MPH310) on a 6.3 mm electrode handle (WPI, 2505) and coupled to a 10 µl microsyringe (Hamilton, 1701LT) using an extension tube. Animals were anesthetized by inhalation of 5% isoflurane and pupils were dilated with 0.4% tropicamide. The string-like aggregates were loaded into a micropipette, and the string-like aggregates were transplanted under the retina.

### Transplantation into rabbit eyes

kbl:JW rabbits (9 weeks old) were obtained from Oriental Yeast Industry Co., Ltd. (Tokyo). The animals were anesthetized with an intramuscular injection of 60 mg/kg ketamine and 10 mg/kg xylazine. After routine vitrectomy with posterior vitreous detachment, string-like aggregates were loaded into a 24-gauge indwelling needle cannula (TOP, SS-6) with a custom-made flat-cut 25G blunt needle as the inner core. After producing a topical retinal detachment with a PolyTip cannula 25g/38g (MedOne, 25g/38g), the graft was slowly injected into the detached retinal bleb using a 50 µl microsyringe (Hamilton, 1705LT). A perfluorocarbon liquid (Alcon, 8065900111) was then injected over the detachment to compress and adhere the retina, followed by fluid-gas exchange.

### Results

### Preparation of string-like aggregate form hiPSC-RPE

A scheme for preparing string-like aggregates using a PDMS-based culture device was summarized. First, human iPSC-RPE cells (253G1 line) were suspended in "RPE maintenance medium", "RPE cell sheet medium (F10 medium containing 10% fetal bovine serum)", or "mix medium" containing 10 µM Y-27632, and seeded in each groove. In the sheet medium, the string-like aggregates appeared to be easily disintegrated, but in the maintenance medium and the mix medium, the string-like aggregate structure was well maintained (Figs. 12A and B). Next, different numbers of cells (4.5×10⁵, 1.5×10⁵, 5×10⁴) were seeded, and it was observed that string-like aggregates could be formed under the former two conditions (Fig. 12C). In the Example, hiPSC-RPE seeded with 4.5×10⁵ cells were detached from the groove and loaded into a 24G intravenous cannula.

### Optimization of Y-27632 concentration for string-like aggregates

Next, string-like aggregates formed by 4.5×10⁵ cells and 1.5×10⁵ cells were plated into the wells. String-like aggregates of the starting cell numbers of the both indicate an expansion of RPE cells on non-coated plates from string-like aggregates, and string-like aggregates formed with 1.5×10⁵ cells lost their initial shape faster as compared with the string-like aggregates formed with 4.5×10⁵ cells that still retained most of their initial shape after 2 weeks (Fig. 13A and Fig. 12D). Therefore, the present inventors fixed the starting cell number at 1.5-2×10⁵ cells. When the string-like aggregates (1.5×10⁵ cells) were fragmented, the initial string-like aggregate form was mostly lost (Fig. 13A').

In the present inventors' preliminary experiment (Fig. 14A), higher concentrations of Y-27632 appeared to affect the texture of the string-like aggregates to become tighter. The inventors tested concentrations of Y-27632 of 0, 1, 2, 2.5 and 10 µM in string-like aggregate formation. hiPSC-RPEs (M8 line) were able to form string-like aggregate shape at concentrations above 2 µM, but string-like aggregates were not successfully formed at 0 and 1 µM Y-27632. The string-like aggregates formed with 10 µM Y-27632 were easily detached after being placed on uncoated wells and, as compared with the string-like aggregates formed with 2-2.5 µM Y-27632, took time to attach on the 24-well plate and expansion was delayed (Figs. 14B and C).

Next, the present inventors directly compared string-like aggregates made with either 2.5 µM or 10 µM of Y-27632 (Figs. 13B-G). The string-like aggregates formed with 2.5 µM Y-27632 appeared flat and loose. On the other hand, string-like aggregates formed with 10 µM Y-27632 appeared more dense (Fig. 13B) .

String-like aggregate sections were stained for gap junction marker Z0-1, polar markers Ezrin (apical), laminin, and collagen type IV (base) (Fig. 13C). Under both conditions, the gap junction marker ZO1 was expressed between cells, the base marker laminin was distributed throughout the cell surface, and the apical marker Ezrin appeared localized on the surface of fragmented cell clusters. Another base marker, collagen IV, was not clearly expressed, and cell polarity is not clearly determined in these string-like aggregates.

The string-like aggregates on day 2 (Figs. 13D and E) and day 3 (Figs. 13F and G) were then used to compare cell expansion after plating in uncoated wells between string-like aggregates formed with 2.5 and 10 µM Y-27632. The string-like aggregates formed with 2.5 µM Y-27632 tended to attach to the plate and begin to expand early after placing. On the other hand, the string-like aggregates formed with 10 µM Y-27632 tended to be coil-like and moved around for several days before settling and starting to expand.

### Reproducibility of string-like aggregate formation using another hiPSC-RPE line

The present inventors tested whether another hiPSC line could similarly form string-like aggregates. The present inventors used the 201B7modFucci line to induce RPE differentiation and compared it with hiPSC(M8)-RPE. hiPSC(201B7modFucci)-RPE also formed string-like aggregates like hiPSC(M8)-RPE, but the 201B7modFucci line required a Y-27632 concentration of 5 µM or higher to stably form string-like aggregates (Fig. 15-1A). The optimal Y-27632 concentration for adhesion/expansion assays was 5 µM for hiPSC(201B7modFucci)-string-like aggregates, and the optimal Y-27632 concentration for the desired adhesion/expansion effect varied depending on the cell line used and required optimization (Figs. 14D and E). Cells expanded from string-like aggregates of both hiPSC lines exhibited the characteristic pigmentation and cobblestone appearance of RPE cells and expressed ZO-1 and MITF (Figs. 15-1B and C).Expression of RPE-specific marker genes (BEST1, RPE65, RLBP1) was also confirmed by polymerase chain reaction (PCR) at each stage of hiPSC-RPE before string-like aggregate formation, during string-like aggregate formation, and after expansion from string-like aggregates (Fig. 15-1D). RPE cells were observed to expand from string-like aggregates into a monolayer on the plate (Fig. 15-1E). To see whether expansion from string-like aggregates is accompanied by proliferation of RPE, the total number of cells 14 days after placing string-like aggregates on the plate was compared with the initial cell number in the string-like aggregates formed on day 2 using 2×10⁵ hiPSC-RPE cells. The total number of cells in expanded cells and remaining string-like aggregates was 5.28×10⁵ on average, which is about four times the average cell number in day-2 string-like aggregates (1.36×10⁵). This indicates that RPE proliferation also contributed to cell expansion from string-like aggregates (day 2 string-like aggregates n=4; expansion from hiPSC(M8)-string-like aggregates n=6; expansion from hiPSC(201B7modFucci)-string-like aggregate n=6) (Fig. 15-2F). The present inventors tested the secretion of VEGF and PEDF by the expanded cells and remaining string-like aggregates combined at the same time point (14 days after placing string-like aggregates).

The expanded hiPSC-RPE secreted VEGF and PEDF to a similar level as compared with the cells originally used for forming string-like aggregates. (hiPSC(M8)-RPE n=6 and hiPSC(201B7modFucci)-RPE n=6; RPE cells of both lines before string-like aggregate formation as a control. n=4) (Fig. 15-2G).

### Replacement of RPE by mimetic hiPSC-RPE transplantation into in vitro RPE injury model with cell suspensions or string-like aggregates

To investigate how transplantation of strand-like aggregates or cells can compensate for damaged REE, the present inventors designed an in vitro model of hiPSC-RPE transplantation in an RPE-damaged environment as shown in Fig. 11A. Briefly, hiPSC-RPE (M8 line) cultures in uncoated 24-well plates were treated with MMC to inhibit cell proliferation, and the center of the wells was scratched the next day. The next day, string-like aggregates or suspended cells (201B7modFucci line) were placed or seeded onto the scratched area, respectively, and monitored using Incucyte for filling or replacement by "transplanted" RPE cells (Fig. 11B).

In control wells (unsupplied wells), the pre-existing MMC-treated RPE gradually expanded and migrated, slowly reducing the defect area. Transplanted RPE cells in suspension rapidly occupied the defect area. When using string-like aggregates, the RPE expanded from the string-like aggregates until it came into contact with a pre-existing RPE, but contact inhibition appeared to discontinue cell expansion from string-like aggregates. The average of the defective region and the region occupied by the transplanted RPE was measured on days 1, 7, and 28 after in vitro transplantation and quantitatively shown in Fig. 11B' (string-like aggregates and suspensions; n=5, control; n=3). In string-like aggregates, the border between pre-existing RPE and transplanted RPE was reasonably identified by the expression of mCherry by transplanted 201B7modFucci cells and the size of RPE cells; MMC-treated, nondividing RPE was generally larger than that expanded from the transplanted RPE (Fig. 11C). The gap junction marker ZO-1 was interestingly observed throughout both the pre-existing and transplanted RPE populations without interfering across the border of the two populations (Fig. 11D).

Furthermore, the secretion of VEGF (Fig. 11E) and PEDF (Fig. 11F) was also monitored as parameters to estimate the overall RPE function in each well. VEGF secretion decreased significantly after scratching, and then increased in all groups until 28 days later. In cell suspension transplantation, VEGF secretion was almost the same as that before MMC treatment. In the transplant group, PEDF secretion decreased after scratching. In string-like aggregate transplantation, PEDF secretion once decreased after scratching, but after 28 days there was no difference from that before MMC treatment. In cell transplantation, PEDF secretion increased significantly after scratching, but without cell replacement, PEDF secretion continued to decrease (control n=4, string-like aggregate n=8, RPE cell n=7).

### String-like aggregate transplantation into nude rats

Next, the present inventors transplanted string-like aggregates formed from 2×10⁵ cells with 2.5 µM or 10 µM Y-27632 into albino nude rats, and tested the operability during surgery and the survival rate of the grafts after transplantation. The transplanted string-like aggregates were successfully transplanted stably subretinally (n=10, 10 µM Y-27632, n=11, 2.5 µM Y-27632) onto the normal RPE of the host eye. The presence of healthy RPE appeared to inhibit the expansion of transplanted string-like aggregates. However, the string-like aggregates survived stably for up to 10 months without unexpected tumor formation or graft loss (Fig. 16A and Table 3).

Cross-sectional views by an optical coherence tomography imaging method (Envisu R2200 VHR, Bioptigen, Inc.) showed clear RPE-like reflections on the graft surface (Fig. 16B). Histological analysis of the same eye revealed that the pigment cell layer of the string-like aggregates was positive for the human marker Ku80/XRCC5 (Figs. 16C and D). These cells clearly expressed apical (Ezrin) and base (collagen type IV) markers and exhibited correct polarity after implantation (Fig. 16E).

**[Table 3]**

| | | observation period | | | |
|---|---|---|---|---|---|
| concentraiton of Y-27632 | cell number to form a strip | 1M | 2M | 3M | 8-10M |
| 10 *µ*M | 200000 | 3 | 2 | 2 | 3 |
| 2.5 *µ*M | 200000 | 3 | 2 | 2 | 4 |

### String-like aggregate injection into rabbit eyes

Finally, the practicality of the surgical technique for string-like aggregate transplantation in clinical practice was confirmed using two rabbit eyes. A 24G intravenous cannula was loaded with string-like aggregates (Fig. 17). After normal vitrectomy, retinal detachment was performed, and string-like aggregates were successfully injected into the retinal hemorrhage area under good visual control (Fig. 18).

### Example 2

Production of a COP-based culture device for production of string-like aggregates of hiPSC-RPE

A cycloolefin polymer (COP) plate (manufactured by Nippon Zeon Co., Ltd.) was cut and processed to produce a COP-based culture device with grooves of length 20 mm, width 1 mm, depth 2 mm (groove bottom radius 0.5 mm) (Fig. 19).

### Preparation of hiPSC-derived RPE cells (hiPSC-RPE)

The M8 hiPSC line (RIKEN) was induced to differentiate using the SFEBq method, and the QHJI01s04 hiPSC line (Kyoto University) was induced to differentiate using the method described in N Engl J Med 2017; 376:1038-46, to respectively prepare hiPSC-derived RPE cells (hiPSC-RPE). The cells were stocked at -150°C in the same manner as in Example 1.

### Production of string-like aggregate

After collecting hiPSC-RPE cells (M8 line, QHJI01s04 line) that had been cultured for 2 weeks after thawing, they were seeded in each groove of a COP-based culture device at a cell number of 2×10⁵ with 10 µl maintenance medium containing 5 µM Y-27632. It was confirmed that string-like aggregates were formed after 2 days.

### Production of string-like aggregates using cells immediately after thawing

hiPSC-RPE cells immediately after thawing were seeded into each groove of a COP-based culture device, and string-like aggregates could be formed after 2 days. Therefore, it became possible to shorten the time from thawing of cells to formation of string-like aggregates to two days.

### Cryopreservation of string-like aggregates

The medium containing the string-like aggregates after production was replaced with STEMCELLBANKER, placed in a cryotube, temporarily stored at -80°C, and then cryopreserved at -150°C. After 4 months, the string-like aggregates were thawed, suspended in a mix medium, and plated onto dishes. It was confirmed that the plated string-like aggregates adhered to the dish and expanded.

### Transplantation into rabbit or monkey eyes

It was confirmed that string-like aggregates could be aspirated and discharged using 25g/31g, 25g/33g, and 25g/38g PolyTip cannulas (MedOne). When a viscoelastic substance (shell gun, viscoat, etc.) diluted 4-7 times with OptiMEM was used as a medium, string-like aggregates could be smoothly aspirated or discharged (Fig. 20). Using these, it was confirmed that a detachment could be actually created under the retina of rabbits and monkeys using a 38g MedOne cannula, and then string-like aggregates could be inserted using 25g/31g and 25g/33g cannulas. Similarly, after creating a detachment in a monkey eyeball, a 20 mm long string-like aggregate of hiPSC-RPE cells was aspirated with about 2 µl of medium, inserted into the retinal detachment site, and engraftment of the string-like aggregate was also confirmed in the funfus oculi of the eye after surgery by funfus oculi observation (Fig. 21).

### [Industrial Applicability]

According to the present invention, it is possible to produce a transplant material of a retinal pigment epithelial cell that can be prepared in a short time at a low cost, and that can be easily controlled at the site of transplantation even though the transplantation method is non-invasive. This application is based on a patent application No. 2021-078154 filed in Japan (filing date: April 30, 2021), the contents of which are incorporated in full herein.

### [Explanation of symbols]

100: device
110: base member
110a: top surface of base member
111: first base part
112: second base part
120: groove
121: cavity part of groove
122: top part of groove
130: boundary line between groove and top surface of base member
200: forming mold
210: mold substrate
210a: one surface of mold substrate
220: protrusion
230: coating layer
240: V-shape convex mold
a1: retinal pigment epithelial cell
A1: string-like aggregate
b1: cavity part depth
B1: entire groove depth
d1: outer diameter of string-like aggregate body
L1: total length of string-like aggregate
W1: width of cavity part
W2: width of opening of groove
X1: cutting line extending in width direction of groove
θ1: angle of inclination of inner wall of groove

## Claims

1. A string-like aggregate of retinal pigment epithelial cells.

2. The string-like aggregate according to claim 1, having a ratio of total length/outer diameter of body of 2 to 1,000.

3. The string-like aggregate according to claim 2, having a circular or ellipse shape in the cross sectional shape.

4. The string-like aggregate according to any one of claims 1 to 3, wherein the retinal pigment epithelial cell is a cell induced to differentiate from a pluripotent stem cell.

5. A device for producing the string-like aggregate according to any one of claims 1 to 3, comprising a base member, wherein the base member has a top surface provided with one or more grooves, and the groove has a cavity part with a length and a width of the string-like aggregate, as a mold for culturing retinal pigment epithelial cells to be seeded into a string-like aggregate.

6. The device according to claim 5, wherein the groove has a cavity part as the bottom of the groove, and a top part located on the cavity part, wherein the top part has an opening of the groove.

7. The device according to claim 5, wherein the groove is V-shaped in the cross sectional shape, and the deepest part of the V-shape is rounded.

8. A method for producing a string-like aggregate of retinal pigment epithelial cells, comprising the following steps:
(1) a step of seeding retinal pigment epithelial cells suspended in a liquid medium in the groove of the device according to claim 5,
(2) a step of culturing the retinal pigment epithelial cells in the groove to form a string-like aggregate of the retinal pigment epithelial cells.

9. The method according to claim 8, wherein the medium comprises a ROCK inhibitor.

10. The method according to claim 9, wherein the ROCK inhibitor is Y-27632.

11. The method according to any one of claims 8 to 10, wherein the retinal pigment epithelial cells cultured in the groove has a density of 2.5×10³ cells/mL to 5×10⁵ cells/mL.

12. The method according to any one of claims 8 to 10, wherein the retinal pigment epithelial cells are cultured for a period of one day to 7 days.

13. A pharmaceutical composition comprising the string-like aggregate of retinal pigment epithelial cells according to any one of claims 1 to 3.

14. A therapeutic drug for a disease caused by a disorder of a retinal pigment epithelium, comprising the string-like aggregate of retinal pigment epithelial cells according to any one of claims 1 to 3.
